# EUROPEAN PATENT APPLICATION

(11) **EP 4 843 584 A1**
(43) Date of publication of application: **25.11.2026**
(21) Application number: 25741483.9
(22) Date of filing: 14.01.2025
(51) Int. Cl.: A61K 38/56, A61K 38/28, A61K 38/26, A61K 38/17, C07K 14/81, A61K 47/26, A61P 3/10

(54) **POLYPEPTIDE COMPOSITION USING ISOLATED BBI AND KTI AND USE THEREOF**

(30) Priority: 15.01.2024 CN 202410057795
(71) Applicant: Hefei Tianhui Biotech Co., Ltd., Hefei, Anhui 230601 (CN)
(72) Inventor: NING, Guang, Hefei, Anhui 230601 (CN); WANG, Weiqing, Hefei, Anhui 230601 (CN); GAO, Xiaoming, Hefei, Anhui 230601 (CN); LV, Hui, Hefei, Anhui 230601 (CN); SUN, Lei, Hefei, Anhui 230601 (CN); ZHANG, Lei, Hefei, Anhui 230601 (CN); XU, Ping, Hefei, Anhui 230601 (CN); CHEN, Shuhao, Hefei, Anhui 230601 (CN); PANG, Di, Hefei, Anhui 230601 (CN); GU, Chunmeng, Hefei, Anhui 230601 (CN); WANG, Yuchan, Hefei, Anhui 230601 (CN)
(74) Representative: Flesselles, Bruno F.G.
(86) International application number: PCT/CN2025/072251
(87) International publication number: WO 2025/152922

(57) **Abstract**

The present invention provides a polypeptide composition for oral administration, wherein the polypeptide composition comprises a polypeptide, an isolated BBI, an isolated KTI, fish oil, and at least one absorption promoter. The isolated BBI is mixed with the isolated KTI in a certain ratio, so that an activity ratio of total trypsin inhibitory (total TI) activity to total chymotrypsin inhibitory (total CI) activity in the composition is 0.5:1 to 10:1.

## Description

### FIELD OF THE INVENTION

The present invention belongs to the pharmaceutical field, and specifically relates to a polypeptide composition using an isolated Bowman-Birk trypsin inhibitor (BBI) and a Kunitz trypsin inhibitor (KTI) and uses thereof.

### BACKGROUND OF THE INVENTION

In the course of disease treatment, the high specificity and effectiveness possessed by polypeptide drugs are making them an ideal choice for the treatment of numerous human diseases. With the development of biotechnology, the clinical application of polypeptide drugs is becoming increasingly widespread, such as calcitonin, insulin, and thymosin. Generally, polypeptide drugs can only be administered by injection. However, administration by injection easily leads to side effects such as infection and inflammation, and patients often struggle to overcome a fear of needles and have poor compliance, which reduces their motivation to take medication on schedule.

Chinese Patent Publication No. CN101547702A, published on September 30, 2009, discloses an oral insulin composition that utilizes a soybean trypsin inhibitor.

Soy Bean Trypsin Inhibitors (SBTI) are the main anti-nutritional factors found in soybeans. Currently, Kunitz Trypsin Inhibitor (KTI) and Bowman-Birk Trypsin Inhibitor (BBI) have been isolated. Therein, KTI is a single-headed inhibitor that can inhibit the activity of trypsin, and BBI is a double-headed inhibitor that can simultaneously inhibit the activities of both trypsin and chymotrypsin.

It has been reported that commercially available SBTI preparations produce highly variable results when used in pharmaceutical compositions (CN104244969A).

### SUMMARY OF THE INVENTION

The applicant found that by adjusting the activity ratio of BBI and KTI, a smaller inter-individual difference in the oral administration of the polypeptide composition can be achieved.

There are two methods for extracting the soybean trypsin inhibitor from soybeans. The first method is to directly extract the soybean trypsin inhibitor from soybeansrather than separately obtaining BBI and KTI from soybeans followed by mixing them to obtain the soybean trypsin inhibitor. The second method is to obtain BBI and KTI separately from soybeans, and then mix the BBI and KTI to obtain the soybean trypsin inhibitor. The applicant found that when using an oral composition comprising the soybean trypsin inhibitor obtained by the first method, the inter-individual difference in administration was relatively large. The applicant speculated that better effects could be obtained by separately regulating the activities of BBI and KTI.

Therefore, in one aspect, the present disclosure provides a polypeptide composition, wherein the polypeptide composition comprises:
a polypeptide, an isolated BBI and an isolated KTI, fish oil, and at least one absorption enhancer;
wherein the isolated BBI and the isolated KTI are mixed in a certain ratio, so that an activity ratio of a total trypsin inhibitory activity (total TI) to a total chymotrypsin inhibitory activity (total CI) in the composition is from 0.5:1 to 10:1.

In some embodiments, the activity ratio of the total trypsin inhibitory activity (total TI) to the total chymotrypsin inhibitory activity (total CI) in the polypeptide composition provided by the present disclosure is from 0.6:1 to 10:1.

In some embodiments, the activity ratio of the total trypsin inhibitory activity (total TI) to the total chymotrypsin inhibitory activity (total CI) in the polypeptide composition provided by the present disclosure is from 0.7:1 to 10:1.

In some embodiments, the activity ratio of the total trypsin inhibitory activity (total TI) to the total chymotrypsin inhibitory activity (total CI) in the polypeptide composition provided by the present disclosure is from 0.8:1 to 10:1.

In some embodiments, the activity ratio of the total trypsin inhibitory activity (total TI) to the total chymotrypsin inhibitory activity (total CI) in the polypeptide composition provided by the present disclosure is from 0.7:1 to 6:1.

In some embodiments, the activity ratio of the total trypsin inhibitory activity (total TI) to the total chymotrypsin inhibitory activity (total CI) in the polypeptide composition provided by the present disclosure is from 0.8:1 to 6:1.

In some embodiments, the activity ratio of the total trypsin inhibitory activity (total TI) to the total chymotrypsin inhibitory activity (total CI) in the polypeptide composition provided by the present disclosure is from 0.9:1 to 6:1.

In some embodiments, the activity ratio of the total trypsin inhibitory activity (total TI) to the total chymotrypsin inhibitory activity (total CI) in the polypeptide composition provided by the present disclosure is from 0.91:1 to 6:1.

In some embodiments, the activity ratio of the total trypsin inhibitory activity (total TI) to the total chymotrypsin inhibitory activity (total CI) in the polypeptide composition provided by the present disclosure is from 0.91:1 to 5.27:1.

In some embodiments, the activity ratio of the total trypsin inhibitory activity (total TI) to the total chymotrypsin inhibitory activity (total CI) in the polypeptide composition provided by the present disclosure is 0.91:1, 1.77:1, 2.16:1, 5.27:1, or a range between any two of the ratios.

In some embodiments, the activity ratio of the total trypsin inhibitory activity (total TI) to the total chymotrypsin inhibitory activity (total CI) in the polypeptide composition provided by the present disclosure is from 0.91:1 to 5.27:1, from 0.91:1 to 2.16:1, from 0.91:1 to 1.77:1, from 1.50:1 to 5.27:1, from 1.60:1 to 5.27:1, from 1.77:1 to 5.27:1, from 1.77:1 to 2.16:1, or from 2.16:1 to 5.27:1.

There are two methods for extracting the soybean trypsin inhibitor from soybeans. The first method is to directly extract the soybean trypsin inhibitor from soybeans (rather than separately obtaining BBI and KTI from soybeans followed by mixing them to obtain the soybean trypsin inhibitor). The second method is to separately obtain BBI and KTI from soybeans, and then mix them to obtain the soybean trypsin inhibitor. The applicant found that when using an oral composition comprising the soybean trypsin inhibitor obtained by the first method, the inter-individual difference in administration was relatively large. The applicant found that better effects could be obtained by separately regulating the activities of BBI and KTI.

In some embodiments, the activity ratio of the total trypsin inhibitory activity (total TI) to the total chymotrypsin inhibitory activity (total CI) in the polypeptide composition provided by the present disclosure is 0.5:1, 0.6:1, 0.7:1, 0.8:1, 0.9:1, 1:1, 2:1, 3:1, 4:1, 5:1, 6:1, 7:1, 8:1, 9:1, 10:1, or a range between any two of the ratios thereof.

In some embodiments, the isolated BBI and the isolated KTI are prepared by the method described in Chinese Patent Application CN104244969, the entire contents of this patent application are incorporated herein by reference.

The amino acid sequence of BBI is as shown in SEQ ID NO: 1, and its accession number in the Uniprot database is UniprtoKB-P01055;

SEQ ID NO: 1
MVVLKVCLVL LFLVGGTTSA NLRLSKLGLL MKSDHQHSND DESSKPCCDQ
CACTKSNPPQ CRCSDMRLNS CHSACKSCIC ALSYPAQCFC VDITDFCYEP
CKPSEDDKEN

As used herein, KTI refers to KTI3. The amino acid sequence of KTI3 is as shown in SEQ ID NO: 2, and the accession number of KTI3 in the Uniprot database is UniprtoKB-P01070.

SEQ ID NO: 2
MKSTIFFLFL FCAFTTSYLP SAIADFVLDN EGNPLENGGT
YYILSDITAF GGIRAAPTGN ERCPLTVVQS RNELDKGIGT IISSPYRIRF
IAEGHPLSLK FDSFAVIMLC VGIPTEWSVV EDLPEGPAVK IGENKDAMDG
WFRLERVSDD EFNNYKLVFC PQQAEDDKCG DIGISIDHDD
GTRRLVVSKN KPLVVQFQKL DKESLAKKNH GLSRSE

As used herein, the term "isolated BBI" refers to a preparation enriched in BBI relative to other components. In various embodiments, the BBI preparation utilized in the methods and polypeptide compositions is at least 85% pure, as determined by SDS-PAGE, brilliant blue staining, or an imager quantitative method. In the context of a polypeptide composition, these values relate to the characteristics of the BBI prior to mixing the BBI with one or more other components of the polypeptide composition. In other embodiments, the BBI preparation contains 5% or less KTI. In other embodiments, the BBI preparation contains less than 0.1% high molecular weight impurities (in other words, high molecular weight impurities refer to substances with a molecular weight greater than 30,000).

As used herein, the term "isolated KTI" refers to a preparation enriched in KTI relative to other components. In various embodiments, the KTI preparation utilized in the methods and polypeptide compositions is at least 85% pure, as determined by SDS-PAGE, brilliant blue staining, or an imager quantitative method. In other embodiments, the protein content of the KTI preparation is greater than 85%, as measured by a BCA assay. In the context of a polypeptide composition, these values relate to the characteristics of the KTI prior to mixing the KTI with one or more other components of the polypeptide composition. In other embodiments, the KTI preparation contains 5% or less BBI, as determined by SDS-PAGE. In other embodiments, the KTI preparation contains less than 0.1% high molecular weight impurities (in other words, high molecular weight impurities refer to substances with a molecular weight greater than 30,000).

In other embodiments, the isolated BBI and the isolated KTI can be isolated from natural sources by means well known to those skilled in the art.

In some embodiments, the isolated BBI is isolated from a soybean product, wherein the isolated BBI has been purified to a purity of at least 85% as measured by sodium dodecyl sulfate polyacrylamide gel electrophoresis (SDS-PAGE); preferably, the BBI has been purified to a purity of at least 86%, at least 87%, at least 88%, at least 89%, at least 90%, or at least 91%;
wherein the isolated BBI has been purified to a protein content of at least 85% as measured by a BCA (Bicinchoninic acid) assay; preferably, the BBI has been purified to a protein content of at least 86%, at least 87%, at least 88%, at least 89%, at least 90%, at least 91%, or at least 92%.

In some embodiments, the isolated BBI contains less than 0.1% high molecular weight impurities.

The isolated KTI is isolated from a soybean product, wherein the isolated KTI has been purified to a purity of at least 85% as measured by sodium dodecyl sulfate polyacrylamide gel electrophoresis (SDS-PAGE); preferably, the KTI has been purified to a purity of at least 86%, at least 87%, at least 88%, at least 89%, at least 90%, or at least 95%;
wherein the isolated KTI has been purified to a protein content of at least 85% as measured by a BCA (Bicinchoninic acid) assay; preferably, the KTI has been purified to a protein content of at least 86%, at least 87%, at least 88%, at least 89%, or at least 90%.

In some embodiments, the isolated KTI contains less than 0.1% high molecular weight impurities.

The purity of the isolated BBI and/or the isolated KTI as described herein is determined prior to mixing with one or more other components of the polypeptide composition.

In embodiments of the present disclosure, the isolated BBI has activity of inhibiting both trypsin and chymotrypsin.

In embodiments of the present disclosure, the isolated BBI has a trypsin inhibitory activity of at least 0.5, 0.6, 0.7, 0.8, or 0.9 mg/mg; and the isolated BBI has a chymotrypsin inhibitory activity of at least 0.5, 0.6, 0.7, 0.8, 0.9, 1.0, 1.1, 1.2, 1.3, 1.4, 1.5, 1.6, 1.7, or 1.8 mg/mg.

In embodiments of the present disclosure, the isolated BBI has a trypsin inhibitory activity of 0.9 mg/mg; and the isolated BBI has a chymotrypsin inhibitory activity of 1.5 mg/mg.

In embodiments of the present disclosure, the isolated KTI has a trypsin inhibitory activity.

In embodiments of the present disclosure, the isolated KTI has a trypsin inhibitory activity of at least 0.5, 0.6, 0.7, 0.8, 0.9, 1.0, 1.1, 1.2, 1.3, 1.4, 1.5, 1.6, 1.7, or 1.8 mg/mg.

In embodiments of the present disclosure, the isolated KTI has a trypsin inhibitory activity of 1.4 mg/mg.

Unless otherwise specified, the chymotrypsin inhibitory activity referred to herein is measured using chymotrypsin having a chymotrypsin activity of 40 BTEE units/mg, and is expressed as the mg of chymotrypsin inhibited per mg of test protein (mg of inhibited chymotrypsin/mg of test protein). BTEE refers to N-benzoyl-L-tyrosine ethyl ester (see Sigma-Aldrich Product Specification No. B6125).

Unless otherwise specified, the trypsin inhibitory activity referred to herein is measured using trypsin having a trypsin activity of 10,000 BAEE units/mg, and is expressed as the mg of trypsin inhibited per mg of test protein (mg of inhibited trypsin/mg of test protein). BAEE refers to N^{α}-benzoyl-L-arginine ethyl ester (see Sigma-Aldrich Product Specification No. B4500).

The chymotrypsin inhibitory activity and the trypsin inhibitory activity as described herein are determined prior to mixing the isolated BBI and/or the isolated KTI with one or more other components of the polypeptide composition.

As used herein, the term "total trypsin inhibitory activity (total TI)" refers to the total trypsin inhibitory activity of BBI and KTI comprised in the polypeptide composition.

As used herein, the term "total chymotrypsin inhibitory activity (total CI)" refers to the chymotrypsin inhibitory activity of BBI comprised in the polypeptide composition.

In some embodiments, the polypeptide composition described herein has a chymotrypsin inhibitory activity that inhibits 0.03 to 0.3 mg of chymotrypsin per mg of polypeptide composition, wherein the chymotrypsin has a chymotrypsin activity of 40 BTEE units/mg.

In some embodiments, the polypeptide composition described herein has a chymotrypsin inhibitory activity that inhibits 0.04 to 0.15 mg of chymotrypsin per mg of polypeptide composition, wherein the chymotrypsin has a chymotrypsin activity of 40 BTEE units/mg.

Specifically, the polypeptide composition described herein has a chymotrypsin inhibitory activity that inhibits at least 0.02, 0.03, 0.04, 0.05, 0.06, 0.07, 0.08, 0.09, 0.1, 0.15, 0.16, 0.17, 0.18, 0.19, 0.2, 0.21, 0.22, 0.23, 0.24, 0.25, 0.26, 0.27, 0.28, 0.3 mg of chymotrypsin per mg of polypeptide composition, wherein the chymotrypsin has a chymotrypsin activity of 40 BTEE units/mg.

In some embodiments, the polypeptide composition described herein has a trypsin inhibitory activity that inhibits 0.05 to 0.4 mg of trypsin per mg of polypeptide composition, wherein the trypsin has a trypsin activity of 10000 BAEE units/mg.

In some embodiments, the polypeptide composition described herein has a trypsin inhibitory activity that inhibits 0.1 to 0.3 mg of trypsin per mg of polypeptide composition, wherein the trypsin has a trypsin activity of 10000 BAEE units/mg.

In some embodiments, the polypeptide composition described herein has a trypsin inhibitory activity that inhibits 0.17 to 0.3 mg of trypsin per mg of polypeptide composition, wherein the trypsin has a trypsin activity of 10000 BAEE units/mg.

In some embodiments, the polypeptide composition described herein has a trypsin inhibitory activity that inhibits 0.06 to 0.17 mg of trypsin per mg of polypeptide composition, wherein the trypsin has a trypsin activity of 10000 BAEE units/mg.

Specifically, the polypeptide composition described herein has a trypsin inhibitory activity that inhibits at least 0.05, 0.06, 0.07, 0.08, 0.09, 0.1, 0.15, 0.16, 0.17, 0.18, 0.19, 0.2, 0.21, 0.22, 0.23, 0.24, 0.25, 0.26, 0.27, 0.28, 0.3 mg of trypsin per mg of polypeptide composition.

In some embodiments, a mass ratio of the isolated BBI to the isolated KTI of the polypeptide composition is from 0.1:1 to 10:1;
in some embodiments, the mass ratio of the isolated BBI to the isolated KTI of the polypeptide composition is from 0.1:1 to 5:1;
in some embodiments, the mass ratio of the isolated BBI to the isolated KTI of the polypeptide composition is from 0.2:1 to 3:1;
in some embodiments, the mass ratio of the isolated BBI to the isolated KTI of the polypeptide composition is from 0.8:1 to 3:1;
in some embodiments, the mass ratio of the isolated BBI to the isolated KTI of the polypeptide composition is from 0.2:1 to 0.8:1;
optionally, the mass ratio of the isolated BBI to the isolated KTI is 0.1:1, 0.2:1, 0.3:1, 0.4:1, 0.5:1, 0.6:1, 0.7:1, 0.8:1, 0.9:1, 1:1, 1.5:1, 2:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1, 10:1, or any numerical value between any two of the ratios.

As used herein, the term "fish oil" is also known as ω-3 fatty acid triglycerides, and its English name is Omega-3-acid triglycerides, which is an oil extracted from fatty fish and is rich in various n-3 polyunsaturated fatty acids such as eicosapentaenoic acid (EPA) and docosahexaenoic acid (DHA). Fatty fish that can be used to extract fish oil include, but are not limited to: mackerel, tuna, salmon, sturgeon, anchovies, sardines, herring, and trout.

In some embodiments, the polypeptide of the present disclosure includes, but is not limited to, any one or a mixture of several selected from the group consisting of insulin, glucagon, Glucagon-like peptide-1 (GLP-1) receptor agonist, Glucagon-like peptide-2 (GLP-2) receptor agonist, Calcitonin (CT), growth hormone, somatostatin, thyrotropin-releasing hormone, parathyroid hormone, gonadotropin-releasing hormone, heparin, granulocyte colony-stimulating factor, prostaglandin, cyclosporine, interferon, vasopressin, vancomycin, erythropoietin, glutathione, and thymosin.

In some embodiments, the polypeptide of the present disclosure includes, but is not limited to, any one or a mixture of several selected from the group consisting of recombinant human insulin, insulin degludec, glucagon, exenatide, benaglutide, liraglutide, loxenatide, dulaglutide, lixisenatide, semaglutide, albiglutide, dulaglutide, teduglutide, calcitonin, salmon calcitonin, growth hormone, octreotide, thyrotropin-releasing hormone, parathyroid hormone fragment, teriparatide, leuprorelin, protirelin, gonadorelin, goserelin, buserelin, sermorelin, nafarelin, histrelin, triptorelin, tesamorelin, corticorelin, lanreotide acetate, terlipressin, antide, teriparatide, abaloparatide, nesiritide, eptifibatide, lixisenatide, sifuvirtide, atosiban, Trametes polysaccharopeptide, ginseng glycopeptide, bone peptide, carnosine and adenosine, glutathione, mannatide, enfuvirtide, bradykinin, enkephalin, nosiheptide, hirudin, glatiramer acetate, aprotinin, alarelin, pancreatic kallikrein, polymyxin, serrapeptase, thymosin α1, thymopentin, and thymalfasin.

In some embodiments, the insulin of the present disclosure includes human insulin, proinsulin, and insulin-like compounds, wherein the insulin-like compounds include insulin analogs and insulin derivatives, etc.

In some embodiments, the insulin of the present disclosure includes animal insulin and human insulin.

In some embodiments, the insulin of the present disclosure includes rapid-acting insulin, short-acting insulin, intermediate-acting insulin, long-acting insulin, and ultra-long-acting insulin.

In some embodiments, the insulin analog of the present disclosure refers to insulin in which one or more amino acids are substituted while retaining some or all of the one or more glucose-related activities of insulin.

In some embodiments, the insulin of the present disclosure includes: (1) rapid-acting insulin, such as insulin lispro, insulin aspart; (2) short-acting insulin, such as recombinant human insulin; (3) intermediate-acting insulin, such as neutral protamine Hagedorn (NPH) insulin; (4) long-acting insulin, such as protamine zinc insulin, insulin glargine, insulin detemir, insulin degludec; (5) ultra-long-acting insulin; (6) shifted insulin analogs; (7) deleted insulin analogs; (8) derivatized insulin; (9) derivatized insulin analogs; (10) derivatized proinsulin; (11) human insulin analog complexes (*e.g.*, hexamer complexes); (12) insulin mixtures; and (13) PEGylated-insulin.

In some embodiments, the glucagon-like peptide-1 of the present disclosure includes short-acting GLP-1 receptor agonists and long-acting GLP-1 receptor agonists.

In some embodiments, the glucagon-like peptide-1 of the present disclosure includes: Exenatide (trade name Byetta), Lixisenatide, Benaglutide (trade name Yishengtai), Liraglutide (trade name Victoza), Taspoglutide, Loxenatide (trade name Fulaimei), Semaglutide, Albiglutide, and Dulaglutide (trade name Trulicity), etc.

In some embodiments, the thymosin of the present disclosure includes: thymosin α1, thymalfasin, thymosin β4, or thymopentin.

In some embodiments, the polypeptide in the composition of the present disclosure can alternatively be replaced with other active substances, such as other small molecule substances with poor oral bioavailability.

In some embodiments, a molecular weight of the polypeptide of the present disclosure is 100 Da to 20000 Da. In some embodiments, the molecular weight of the polypeptide of the present disclosure can be 100 Da, 200 Da, 300 Da, 400 Da, 500 Da, 600 Da, 700 Da, 800 Da, 900 Da, 1000 Da, 1100 Da, 1200 Da, 1300 Da, 1400 Da, 1500 Da, 1600 Da, 1700 Da, 1800 Da, 1900 Da, 2000 Da, 2100 Da, 2200 Da, 2300 Da, 2400 Da, 2500 Da, 2600 Da, 2700 Da, 2800 Da, 2900 Da, 3000 Da, 3100 Da, 3200 Da, 3300 Da, 3400 Da, 3500 Da, 3600 Da, 3700 Da, 3800 Da, 3900 Da, 4000 Da, 4100 Da, 4200 Da, 4300 Da, 4400 Da, 4500 Da, 4600 Da, 4700 Da, 4800 Da, 4900 Da, 5000 Da, 5100 Da, 5200 Da, 5300 Da, 5400 Da, 5500 Da, 5600 Da, 5700 Da, 5800 Da, 5900 Da, 6000 Da, 6100 Da, 6200 Da, 6300 Da, 6400 Da, 6500 Da, 6600 Da, 6700 Da, 6800 Da, 6900 Da, 7000 Da, 7100 Da, 7200 Da, 7300 Da, 7400 Da, 7500 Da, 7600 Da, 7700 Da, 7800 Da, 7900 Da, 8000 Da, 8100 Da, 8200 Da, 8300 Da, 8400 Da, 8500 Da, 8600 Da, 8700 Da, 8800 Da, 8900 Da, 9000 Da, 9100 Da, 9200 Da, 9300 Da, 9400 Da, 9500 Da, 9600 Da, 9700 Da, 9800 Da, 9900 Da, 10000 Da, 11000 Da, 12000 Da, 13000 Da, 14000 Da, 15000 Da, 16000 Da, 17000 Da, 18000 Da, 19000 Da, 20000 Da; or any range between the above numerical values.

In some embodiments, the molecular weight of the polypeptide of the present disclosure is 1000 Da to 20000 Da. In some embodiments, the molecular weight of the polypeptide of the present disclosure is 1000 Da to 6150 Da. In some embodiments, the molecular weight of the polypeptide of the present disclosure is 1209 Da to 6104 Da. In some embodiments, the molecular weight of the polypeptide of the present disclosure is 1000 Da to 6000 Da. In some embodiments, the molecular weight of the polypeptide of the present disclosure is 2000 Da to 6000 Da.

In some embodiments, the polypeptide of the present disclosure can be selected from the following polypeptides:

| No. | Name | English name | Molecular weight |
|---|---|---|---|
| 1 | Leuprorelin | Leuprorelin | 1209.4 |
| 2 | Thymalfasin | Thymalfasin | 3108.3 |
| 3 | Liraglutide | Liraglutide | 3751.0 |
| 4 | Exenatide | Exenatide | 4187.0 |
| 5 | Abaloaratide | Abaloaratide | 3960.59 |
| 6 | Semaglutide | Semaglutide | 4113.58 |
| 7 | Teriparatide | Teriparatide | 4177.77 |
| 8 | Human Insulin | Insulin | 5807.69 |
| 9 | Growth Hormone | Human Growth Hormone, hGH | 22125 |
| 10 | Insulin Degludec | Insulin Degludec | 6103.97 |

In some embodiments, the polypeptide of the present disclosure can be a naturally extracted polypeptide, a chemically synthesized polypeptide, or a polypeptide prepared by genetic engineering.

In some embodiments, the human insulin of the present disclosure can be insulin prepared by a chemical synthesis method, or recombinant human insulin obtained using genetic engineering.

In some embodiments, the polypeptide composition of the present disclosure is for oral administration. In some embodiments, the polypeptide composition for oral administration of the present disclosure is formulated into the following dosage forms: tablet, pill, capsule, emulsion, syrup, or suspension.

Compared with the nanosystems prepared using existing technologies, the polypeptide composition system provided by the present disclosure features the polypeptide in the form of a suspension, the system is homogeneous and exhibits no burst release. Additionally, since encapsulation efficiency is not a concern during the preparation process, product quality is easily controlled.

In some embodiments, the polypeptide composition system provided by the present disclosure is a non-nanosystem.

In some embodiments, the polypeptide composition provided by the present disclosure is not an oil-in-water or water-in-oil system.

In some embodiments, the absorption enhancer of the present disclosure is selected from the group consisting of any one or more of ethylenediaminetetraacetic acid or a salt thereof, ethylene glycol-bis(2-aminoethylether)-N,N,N',N'-tetraacetic acid or a salt thereof, N-(8-(2-hydroxybenzoyl)amino)caprylic acid or a salt thereof, salicylic acid or a salt thereof, citric acid or a salt thereof, cholic acid or a salt thereof, deoxycholic acid or a salt thereof, glycocholic acid or a salt thereof, taurocholic acid or a salt thereof, alkyl glycoside, sodium dodecyl sulfate, docusate sodium, cyclodextrin or a derivative thereof, chitosan or a derivative thereof, caprylic acid or a salt thereof, capric acid or a salt thereof, lauric acid or a salt thereof, myristic acid, palmitic acid, stearic acid, fatty acid triglyceride, lecithin, choline, and carnitine. In some embodiments, the absorption enhancer of the present disclosure is selected from the group consisting of sodium salts of ethylenediaminetetraacetic acid.

In some embodiments, the ethylenediaminetetraacetic acid salt of the present disclosure is selected from the group consisting of disodium ethylenediaminetetraacetate, dipotassium ethylenediaminetetraacetate, trisodium ethylenediaminetetraacetate, or tetrasodium ethylenediaminetetraacetate.

In some embodiments, the polypeptide composition of the present disclosure further comprises at least one emulsifier.

In some embodiments, in the polypeptide composition of the present disclosure, the emulsifier is selected from the group consisting of any one or more of an ionic surfactant, a nonionic surfactant, and a zwitterionic surfactant;
preferably, the emulsifier is selected from the group consisting of any one or more of ammonium lauryl sulfate, N-dodecyl-β-D-maltoside, tridecyl-β-D-maltoside, sodium dodecyl sulfate, docusate sodium, cyclodextrin or a derivative thereof, chitosan or a derivative thereof, sodium polyoxyethylene alkyl sulfate, sodium laureth sulfate, sodium dioctyl sulfosuccinate, poloxamer, glyceryl monostearate, glyceryl monolaurate, sorbitan monolaurate, sorbitan monostearate, polyoxyethylene sorbitan fatty acid ester, sorbitan fatty acid ester, polyoxyethylene monopalmitate, polyoxyethylene hydrogenated castor oil, C6-C12 fatty acid triglyceride, and C8-C10 fatty acid triglyceride;
preferably, the at least one surfactant is selected from Tween; preferably, the at least one surfactant is selected from the group consisting of any one or more of Tween 20, Tween 40, Tween 60, Tween 65, Tween 80, and Tween 85.

In some embodiments, the present disclosure provides a polypeptide composition comprising by mass fraction:
polypeptide: 0.5-10%,
fish oil: 3.0-50.0%,
isolated BBI: 1.0-25.0%,
isolated KTI: 1.0-20.0%,
absorption enhancer: 5.0-45.0%, and
emulsifier: 8.0-60.0%;
   or
polypeptide: 0.9-4.6%,
fish oil: 5.5-34.9%,
isolated BBI: 2.9-10.0%,
isolated KTI: 2.5-14.3%,
absorption enhancer: 12.9-36.1%, and
emulsifier: 14.9-49.6%;
   or
polypeptide: 0.9-4.6%,
fish oil: 11.9-34.9%,
isolated BBI: 2.9-10.0%,
isolated KTI: 2.5-14.3%,
absorption enhancer: 12.9-36.1%, and
emulsifier: 14.9-34.5%;
   or
polypeptide: 0.9-4.6%,
fish oil: 34.5-34.9%,
isolated BBI: 2.9-7.5%,
isolated KTI: 2.5-14.3%,
absorption enhancer: 12.9-35.6%, and
emulsifier: 14.9-34.5%;
   or
polypeptide: 0.9-1.8%,
fish oil: 11.9-34.5%,
isolated BBI: 2.9-10.0%,
isolated KTI: 12.6-14.3%,
absorption enhancer: 12.9-36.1%, and
emulsifier: 27.6-34.5%;
   or
polypeptide: 1.8-4.6%,
fish oil: 11.9-34.9%,
isolated BBI: 7.5-10.0%,
isolated KTI: 2.5-12.6%,
absorption enhancer: 35.6-36.1%, and
emulsifier: 14.9-27.6%.

In some embodiments, the present disclosure provides a polypeptide composition comprising by mass fraction:
recombinant human insulin: 0.5-10%,
fish oil: 3.0-50.0%,
isolated BBI: 1.0-25.0%,
isolated KTI: 1.0-20.0%,
ethylenediaminetetraacetic acid or a salt thereof: 5.0-45.0%, and
Tween: 8.0-60.0%;
   or
recombinant human insulin: 0.9-4.6%,
fish oil: 5.5-34.9%,
isolated BBI: 2.9-10.0%,
isolated KTI: 2.5-14.3%,
ethylenediaminetetraacetic acid or a salt thereof: 12.9-36.1%, and
Tween: 14.9-49.6%;
   or
recombinant human insulin: 0.9-4.6%,
fish oil: 11.9-34.9%,
isolated BBI: 2.9-10.0%,
isolated KTI: 2.5-14.3%,
ethylenediaminetetraacetic acid or a salt thereof: 12.9-36.1%, and
Tween: 14.9-34.5%;
   or
recombinant human insulin: 0.9-4.6%,
fish oil: 34.5-34.9%,
isolated BBI: 2.9-7.5%,
isolated KTI: 2.5-14.3%,
ethylenediaminetetraacetic acid or a salt thereof: 12.9-35.6%, and Tween: 14.9-34.5%;
   or
recombinant human insulin: 0.9-1.8%,
fish oil: 11.9-34.5%,
isolated BBI: 2.9-10.0%,
isolated KTI: 12.6-14.3%,
ethylenediaminetetraacetic acid or a salt thereof: 12.9-36.1%, and Tween: 27.6-34.5%;
   or
recombinant human insulin: 1.8-4.6%,
fish oil: 11.9-34.9%,
isolated BBI: 7.5-10.0%,
isolated KTI: 2.5-12.6%,
ethylenediaminetetraacetic acid or a salt thereof: 35.6-36.1%, and
Tween: 14.9-27.6%.

As used herein, the term "mass fraction" refers to the mass percentage of each component accounting for the total mass of the polypeptide composition.

For example, in some embodiments, the present disclosure provides a polypeptide composition comprising
recombinant human insulin: 8 mg,
fish oil: 320 mg,
isolated BBI: 26.7 mg,
isolated KTI: 133.3 mg,
ethylenediaminetetraacetic acid or a salt thereof: 120 mg, and
Tween 80: 320 mg.

The total mass of the polypeptide composition is 928 mg, and the mass fraction of recombinant human insulin is 0.9%.

In some embodiments, the present disclosure provides a polypeptide composition comprising in a unit dosage form:
recombinant human insulin: 8-32 mg,
fish oil: 50-320 mg,
isolated BBI: 26.7-88.9 mg,
isolated KTI: 17.5-133.3 mg,
ethylenediaminetetraacetic acid or a salt thereof: 120-320 mg, and
Tween: 105-450 mg;
   or
recombinant human insulin: 8-32 mg,
fish oil: 105-320 mg,
isolated BBI: 26.7-88.9 mg,
isolated KTI: 17.5-133.3 mg,
ethylenediaminetetraacetic acid or a salt thereof: 120-320 mg, and
Tween: 105-320 mg;
   or
recombinant human insulin: 8-32 mg,
fish oil: 245-320 mg,
isolated BBI: 26.7-52.5 mg,
isolated KTI: 17.5-133.5 mg,
ethylenediaminetetraacetic acid or a salt thereof: 120-250 mg, and
Tween: 105-320 mg;
   or
recombinant human insulin: 8-16 mg,
fish oil: 105-320 mg,
isolated BBI: 26.7-88.9 mg,
isolated KTI: 111.1-133.3 mg,
ethylenediaminetetraacetic acid or a salt thereof: 120-320 mg, and
Tween: 245-320 mg;
   or
recombinant human insulin: 8 mg,
fish oil: 50-320 mg,
isolated BBI: 26.7-37.5 mg,
isolated KTI: 62.5-133.3 mg,
ethylenediaminetetraacetic acid or a salt thereof: 120-300 mg, and
Tween: 320-450 mg;
   or
recombinant human insulin: 16-32 mg,
fish oil: 105-245 mg,
isolated BBI: 52.5-88.9 mg,
isolated KTI: 17.5-111.1 mg,
ethylenediaminetetraacetic acid or a salt thereof: 250-320 mg, and
Tween: 105-245 mg;
   or
recombinant human insulin: 8-16 mg,
fish oil: 50-105 mg,
isolated BBI: 37.5-88.9 mg,
isolated KTI: 62.5-111.1 mg,
ethylenediaminetetraacetic acid or a salt thereof: 300-320 mg, and
Tween: 245-450 mg.

In some embodiments, the present disclosure provides a polypeptide composition comprising in a unit dosage form:
recombinant human insulin: 8 mg,
fish oil: 320 mg,
isolated BBI: 26.7 mg,
isolated KTI: 133.3 mg,
ethylenediaminetetraacetic acid or a salt thereof: 120 mg, and
Tween: 320 mg;
   or
recombinant human insulin: 16 mg,
fish oil: 105 mg,
isolated BBI: 88.9 mg,
isolated KTI: 111.1 mg,
ethylenediaminetetraacetic acid or a salt thereof: 320 mg, and
Tween: 245 mg;
   or
recombinant human insulin: 32 mg,
fish oil: 245 mg,
isolated BBI: 52.5 mg,
isolated KTI: 17.5 mg,
ethylenediaminetetraacetic acid or a salt thereof: 250 mg, and
Tween: 105 mg;
   or
recombinant human insulin: 8 mg,
fish oil: 50 mg,
isolated BBI: 37.5 mg,
isolated KTI: 62.5 mg,
ethylenediaminetetraacetic acid or a salt thereof: 300 mg, and
Tween: 450 mg.

In some embodiments, the polypeptide composition described herein comprises a polypeptide, and the mass fraction of the polypeptide can be 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 2%, 3%, 4%, 5%, 6%, 7%, 8%, 9%, 10%, or any range or point value therebetween.

In some embodiments, the polypeptide composition described herein comprises an isolated BBI, and the mass fraction of the isolated BBI can be 1.0%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, or any range or point value therebetween.

In some embodiments, the polypeptide composition described herein comprises an isolated KTI, and the mass fraction of the isolated KTI can be 1.0%, 2%, 3%, 4%, 5%, 6%, 7%, 8%, 9%, 10%, 11%, 12%, 13%, 14%, 15%, or any range or point value therebetween.

In some embodiments, the polypeptide composition described herein comprises an absorption enhancer, and the mass fraction of the absorption enhancer can be 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, or any range or point value therebetween.

In some embodiments, the polypeptide composition of the present disclosure further comprises at least one emulsifier.

In some embodiments, the emulsifier of the present disclosure includes any one or a combination of at least two selected from the group consisting of an ionic surfactant, a nonionic surfactant, and a zwitterionic surfactant.

In some embodiments, the emulsifier of the present disclosure is an ionic surfactant. In some embodiments, the ionic surfactant of the present disclosure includes, but is not limited to, any one or a combination of at least two selected from the group consisting of sulfates, sulfonates, phosphates, carboxylates, ammonium lauryl sulfate, sodium dodecyl sulfate (SDS), sodium polyoxyethylene alkyl sulfate, sodium laureth sulfate, docusate sodium (dioctyl sodium sulfosuccinate, DSS), perfluorooctane sulfonate (PFOS), perfluorobutane sulfonate, alkyl aryl ether phosphates, and alkyl ether phosphates.

In some embodiments, the emulsifier of the present disclosure is a nonionic surfactant. In some embodiments, the nonionic surfactant of the present disclosure includes, but is not limited to, any one or a combination of at least two selected from the group consisting of Triton X-100, poloxamer, glyceryl monostearate, glyceryl monolaurate, sorbitan monolaurate, sorbitan monostearate, polyoxyethylene sorbitan fatty acid ester (*i.e.*, Tweens, such as Tween 20, Tween 40, Tween 60, and Tween 80), and sorbitan fatty acid ester (Span).

The emulsifier of the present disclosure can also be any one or a combination of at least two selected from the group consisting of 1,2-dipalmitoyl-sn-glycero-3-phosphoglycerol (DPPG), 1-palmitoyl-2-oleoyl-sn-glycero-3-phosphoethanolamine (POPE), *N*-dodecyl-β-D-maltoside, tridecyl-β-D-maltoside, cyclodextrin, and chitosan derivatives (*e.g.*, protonated chitosan, trimethyl chitosan chloride, etc.).

In some embodiments, the polypeptide composition further comprises at least one emulsifier; preferably, the polypeptide composition comprises 0.1-75% emulsifier, preferably 1-60%, further preferably 5-50%.

In some embodiments, the polypeptide composition described herein comprises an emulsifier, and the mass fraction of the emulsifier can be 0.1%, 0.2%, 0.3%, 0.4%, 0.5%, 0.6%, 0.7%, 0.8%, 0.9%, 1.0%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, or any range or point value therebetween.

In the above embodiments, the mass ratio of the isolated BBI to the isolated KTI in the polypeptide composition is from 0.1:1 to 10:1; optionally, the mass ratio of the isolated BBI to the isolated KTI is 0.1:1, 0.2:1, 0.3:1, 0.4:1, 0.5:1, 0.6:1, 0.7:1, 0.8:1, 0.9:1, 1:1, 1.5:1, 2:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1, 10:1, or any numerical value between any two of the ratios.

In embodiments of the present disclosure, a polypeptide composition is provided, wherein the isolated BBI and the isolated KTI in the polypeptide composition are mixed in a certain ratio, so that the activity ratio of the total trypsin inhibitory activity (total TI) to the total chymotrypsin inhibitory activity (total CI) in the composition is from 0.5:1 to 10:1; preferably from 0.7:1 to 10:1; preferably from 0.91:1 to 6:1; preferably from 0.91:1 to 5.27:1; and the polypeptide composition comprises by mass fraction:
recombinant human insulin: 0.5-10%,
fish oil: 3.0-50.0%,
isolated BBI: 1.0-25.0%,
isolated KTI: 1.0-20.0%,
ethylenediaminetetraacetic acid or a salt thereof: 5.0-45.0%, and
Tween: 8.0-60.0%;
   or
recombinant human insulin: 0.9-4.6%,
fish oil: 5.5-34.9%,
isolated BBI: 2.9-10.0%,
isolated KTI: 2.5-14.3%,
ethylenediaminetetraacetic acid or a salt thereof: 12.9-36.1%, and
Tween: 14.9-49.6%;
   or
recombinant human insulin: 0.9-4.6%,
fish oil: 11.9-34.9%,
isolated BBI: 2.9-10.0%,
isolated KTI: 2.5-14.3%,
ethylenediaminetetraacetic acid or a salt thereof: 12.9-36.1%, and
Tween: 14.9-34.5%;
   or
recombinant human insulin: 0.9-4.6%,
fish oil: 34.5-34.9%,
isolated BBI: 2.9-7.5%,
isolated KTI: 2.5-14.3%,
ethylenediaminetetraacetic acid or a salt thereof: 12.9-35.6%, and
Tween: 14.9-34.5%;
   or
recombinant human insulin: 0.9-1.8%,
fish oil: 11.9-34.5%,
isolated BBI: 2.9-10.0%,
isolated KTI: 12.6-14.3%,
ethylenediaminetetraacetic acid or a salt thereof: 12.9-36.1%, and
Tween: 27.6-34.5%;
   or
recombinant human insulin: 1.8-4.6%,
fish oil: 11.9-34.9%,
isolated BBI: 7.5-10.0%,
isolated KTI: 2.5-12.6%,
ethylenediaminetetraacetic acid or a salt thereof: 35.6-36.1%, and
Tween: 14.9-27.6%.

In another aspect, the present disclosure also provides a drug comprising the polypeptide composition described above. In some embodiments, the drug of the present disclosure further comprises a pharmaceutically acceptable excipient.

In some embodiments, the pharmaceutically acceptable excipient of the present disclosure comprises a coating.

In some embodiments, the coating of the present disclosure is selected from the group consisting of a gastric-soluble coating and/or an enteric-soluble coating.

In some embodiments, the coating material of the present disclosure includes: hydroxypropyl methylcellulose (HPMC), hydroxypropyl cellulose (HPC), acrylic resin, polyvinylpyrrolidone, cellulose acetate phthalate (CAP), hydroxypropyl methylcellulose phthalate (HPMCP), hydroxypropyl methylcellulose acetate succinate (HPMCAS), etc.

It should be noted that in the present disclosure, the main function of the coating is to allow the polypeptide to be released at a specific site within the gastrointestinal tract; for example, the enteric-soluble coating allows the polypeptide to be insoluble in gastric acid and soluble in intestinal fluid. Those skilled in the art can select the coating material according to actual needs, thereby achieving the release of the polypeptide into gastric fluid or intestinal fluid within the gastrointestinal tract.

In some embodiments, the pharmaceutically acceptable excipients of the present disclosure include diluents, disintegrants, binders, flavoring agents, or lubricants.

In some embodiments, the excipient of the present disclosure is selected from the group consisting of any one or a combination of at least two of lactose, sorbitol, xylitol, mannitol, calcium hydrogen phosphate, microcrystalline cellulose, silicified microcrystalline cellulose, starch, pregelatinized starch, lactose-starch complex, lactose-cellulose complex, or mannitol-starch complex.

In some embodiments, the disintegrant of the present disclosure is selected from the group consisting of any one or a combination of at least two of croscarmellose sodium, cross-linked polyvinylpyrrolidone, sodium starch glycolate, or low-substituted hydroxypropyl cellulose.

In some embodiments, the binder of the present disclosure is selected from the group consisting of any one or a combination of at least two of colloidal microcrystalline cellulose, hydroxypropyl methylcellulose, hydroxypropyl cellulose, methylcellulose, ethylcellulose, polyvinylpyrrolidone, gelatin solution, starch paste, or sucrose solution.

In some embodiments, the lubricant of the present disclosure is selected from the group consisting of any one or a combination of at least two of sodium stearyl fumarate, magnesium stearate, calcium stearate, micronized silica gel, or talc.

In some embodiments, the flavoring agent of the present disclosure is any one or a combination of at least two of mannitol, sorbitol, aspartame, stevioside, sucralose, aspartame, neotame, steviol glycosides, acesulfame chloride, or flavoring essence.

In some embodiments, the polypeptide composition and the drug comprising the same provided by the present disclosure are for oral administration.

In some embodiments, the polypeptide composition or drug provided in the present disclosure includes a tablet, pill, capsule, emulsion, syrup, or suspension; preferably, the polypeptide within the capsule contents is in a suspended state.

In some embodiments, the polypeptide composition or drug provided in the present disclosure is anhydrous.

"Anhydrous" means that the moisture content of the capsule contents is less than 9%. For example, the moisture content can be 8%, 7%, 6%, or 5%. Moisture is determined according to the Karl Fischer method of General Rule 0832 of the Chinese Pharmacopoeia 2020 Edition.

In some embodiments, the polypeptide composition or drug provided in the present disclosure is an oral or injectable dosage form.

In some embodiments, the polypeptide composition or drug provided in the present disclosure is for use in the preparation of an oral drug for treating or preventing a disease, wherein the disease is selected from the group consisting of hyperglycemia, type 1 diabetes, type 2 diabetes, non-insulin-dependent diabetes, maturity-onset diabetes of the young, gestational diabetes, obesity, non-alcoholic fatty liver disease, hepatic steatosis, hepatitis, liver fibrosis, cirrhosis, liver cancer, Alzheimer's disease, Parkinson's disease, and diseases with impaired glucose tolerance.

In some embodiments, the present disclosure provides a method for treating or preventing a disease, the method comprising orally administering to a subject in need thereof a therapeutically effective amount of the polypeptide composition or the drug, wherein the disease is selected from the group consisting of hyperglycemia, type 1 diabetes, type 2 diabetes, non-insulin-dependent diabetes, maturity-onset diabetes of the young, gestational diabetes, obesity, non-alcoholic fatty liver disease, hepatic steatosis, hepatitis, liver fibrosis, cirrhosis, liver cancer, Alzheimer's disease, Parkinson's disease, and diseases with impaired glucose tolerance.

In some embodiments, the present disclosure provides a method for treating or preventing a disease, the method comprising orally administering to a subject in need thereof the polypeptide composition in a single dose of 8 to 50 mg of the polypeptide, once to three times daily; or orally administering to the subject the drug in a single dose of 8 to 50 mg of the polypeptide, once to three times daily.

In some embodiments, the present disclosure provides a method for treating or preventing a disease, the method comprising orally administering to a subject in need thereof the polypeptide composition in a single dose of 8 mg, 24 mg, or 50 mg, once twice, or three times daily; or orally administering to the subject the drug according to any one of claims 24-25 in a single dose of 8 mg, 24 mg, or 50 mg of the polypeptide, once or twice daily.

### DETAILED DESCRIPTION

The present invention is described in detail below in conjunction with the accompanying drawings.

### Example 1 Preparation of Soybean Trypsin Inhibitor

Soy Bean Trypsin Inhibitors (abbreviated as SBTI) are the main anti-nutritional factors in soybeans, mainly including Kunitz trypsin inhibitor (abbreviated as KTI) and Bowman-Birk trypsin inhibitor (abbreviated as BBI). Kunitz trypsin inhibitor (abbreviated as KTI) has trypsin inhibitory activity, and Bowman-Birk trypsin inhibitor (abbreviated as BBI) has both trypsin inhibitory activity and chymotrypsin inhibitory activity.

Trypsin inhibitory activity is abbreviated as "TI", and chymotrypsin inhibitory activity is abbreviated as "CI".

The amino acid sequence of BBI is as shown in SEQ ID NO: 1, and its accession number in the Uniprot database is UniprtoKB-P01055;

SEQ ID NO: 1
MVVLKVCLVL LFLVGGTTSA NLRLSKLGLL MKSDHQHSND
DESSKPCCDQ CACTKSNPPQ CRCSDMRLNS CHSACKSCIC ALSYPAQCFC
VDITDFCYEP CKPSEDDKEN

KTI referred to herein is KTI3, and the amino acid sequence of KTI3 are as shown in SEQ ID NO: 2, and the accession number of KTI3 in the Uniprot database is UniprtoKB-P01070.

SEQ ID NO: 2
MKSTIFFLFL FCAFTTSYLP SAIADFVLDN EGNPLENGGT
YYILSDITAF GGIRAAPTGN ERCPLTVVQS RNELDKGIGT IISSPYRIRF
IAEGHPLSLK FDSFAVIMLC VGIPTEWSVV EDLPEGPAVK IGENKDAMDG
WFRLERVSDD EFNNYKLVFC PQQAEDDKCG DIGISIDHDD GTRRLVVSKN
KPLVVQFQKL DKESLAKKNH GLSRSE

BBI and KTI were prepared by referring to the SBTI preparation method described in Example 2 of Chinese Patent Application No. CN201380018355.7, Patent Publication No. CN104244969A. The patent application is incorporated herein by reference in its entirety.

Briefly, the preparation of BBI and KTI was as described below:
Preparation of SBTI intermediate:
Extraction buffer: 25 mM sodium phosphate and 100 mM sodium chloride, pH 4.5
Dialysis buffer: 10 mM sodium phosphate, pH 6.5
25 kg of soybean flour was mixed with 250 L of the extraction buffer at room temperature for 1 h, and the pH was adjusted to 4.5 with hydrochloric acid. The extraction buffer was 25 mM sodium phosphate and 100 mM sodium chloride, pH 4.5. The mixture was filtered, and the filter cake was washed with the extraction buffer. The filtrate was collected, ammonium sulfate was added to the filtrate, followed by stirring for 1-3 h. The mixture was allowed to stand overnight, and then centrifuged to collect a precipitate. The collected precipitate was mixed with 12 L of 10 mM sodium phosphate buffer (pH 7.6) for 0.5 h, followed by centrifugation to collect a filtrate and a filter cake. The filter cake was repeatedly extracted three more times with the dialysis buffer, and all filtrates were combined. The combined filtrate was dialyzed against the dialysis buffer and centrifuged to collect a filtrate. The filtrate was lyophilized to obtain the SBTI intermediate.

Purification of SBTI intermediate:
The SBTI intermediate was dissolved in a buffer of 10 mM sodium phosphate (pH 6.5). 1 kg of the suspended SBTI intermediate was loaded onto a 30 L column for DEAE-agarose gel column chromatography. Flow rate: 1×CV/30 minutes, and washing was performed with 8×CV of DEAE buffer: 10 mM sodium phosphate, pH 6.5. Elution was performed with a sodium chloride fractional gradient, and a BBI solution and a KTI solution were collected separately. The collected BBI solution and KTI solution were subjected to ultrafiltration desalination and concentration separately to obtain a BBI concentrate and a KTI concentrate, respectively. The BBI concentrate was subjected to two-step ultrafiltration through 30 kDa and 3 kDa ultrafiltration membranes, filtered through a 0.22-micron filter, and lyophilized to obtain BBI. The KTI concentrate was subjected to one-step ultrafiltration concentration and desalination through 10 kDa membrane, filtered through a 0.22-micron filter, and lyophilized to obtain KTI.

### Example 2 Determination of Inhibitory Activity of Soybean Trypsin Inhibitor

Trypsin inhibitory activity and chymotrypsin inhibitory activity were determined for the BBI and KTI obtained in Example 1. The specific determination method and principle were as follows:
Trypsin inhibitory activity determination: measured using trypsin having a trypsin activity of 10,000 BAEE units/mg, and expressed as mg of trypsin inhibited per mg of test protein.

Principle: BAEE was used as a substrate, and the trypsin inhibitory activity of soybean trypsin inhibitor was determined by measuring the absorbance at 253 nm of a reaction mixture containing the substrate (BAEE), trypsin, and the soybean trypsin inhibitor at pH 7.6 and 25°C.

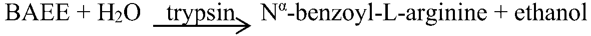

The English name of BAEE is N^{α}-Benzoyl-L-arginine Ethyl Ester.

Specifically,
Unit definition:
Using BAEE as a reaction substrate, under conditions of a pH value of 7.6, a temperature of 25°C, and a reaction volume of 3.20 mL, the consumption of 1 unit of BAEE results in a ΔA253 of 0.001 per minute.

The following reagents were formulated:
a. 67 mM sodium phosphate buffer, pH 7.6, 25°C, adjusted to pH 7.6 with 1 M NaOH at 25°C.
b. 0.25 mM N^{α}-benzoyl-L-arginine ethyl ester solution (BAEE), formulated using the 67 mM sodium phosphate buffer.
c. 1 mM hydrochloric acid solution
d. Trypsin solution, formulated immediately before use with a cold 1 mM hydrochloric acid solution into a solution containing 1 mg of protein per mL of trypsin.
e. Trypsin inhibitor solution (BBI or KTI), formulated immediately before use in cold reagent a into a solution containing 1.0 mg/mL trypsin inhibitor.

Part A:
The following reagents were pipetted (in mL) into 6 centrifuge tubes respectively according to Table 1, and each centrifuge tube was inverted to mix the content uniformly.

**Table 1**

| | Non-inhibited group (mL) | Sample 1 (mL) | Sample 2 (mL) | Sample 3 (mL) | Sample 4 (mL) | Sample 5 (mL) |
|---|---|---|---|---|---|---|
| Reagent e (trypsin inhibitor solution) | - | 0.10 | 0.15 | 0.20 | 0.25 | 0.30 |
| Reagent d (trypsin solution) | 0.50 | 0.50 | 0.50 | 0.50 | 0.50 | 0.50 |
| Reagent c (hydrochloric acid) | 9.50 | 9.40 | 9.35 | 9.30 | 9.25 | 9.20 |

The tubes were allowed to stand at 25°C for at least 5 minutes and no more than 6 minutes.

Part B:
The following reagents were pipetted (in mL) into 7 centrifuge tubes respectively according to Table 2, and each centrifuge tube was inverted to mix the content uniformly.

**Table 2**

| | Non-inhibited group (mL) | Sample 1 (mL) | Sample 2 (mL) | Sample 3 (mL) | Sample 4 (mL) | Sample 5 (mL) | Blank |
|---|---|---|---|---|---|---|---|
| Reagent b (BAEE) | 3.00 | 3.00 | 3.00 | 3.00 | 3.00 | 3.00 | 3.00 |
| Reagent c (HCl) | 0.10 | 0.10 | 0.10 | 0.10 | 0.10 | 0.10 | 0.10 |

Each centrifuge tube was inverted to mix the content and equilibrated to 25°C. A253nm was monitored using a UV-visible spectrophotometer until constant. Reagents of Table 3 were added to Table 2. Specifically, 0.10 mL of the non-inhibited group of Part A was pipetted into the non-inhibited group of Table 2, 0.10 mL of sample 1 of Part A was pipetted into sample 1 of Table 2, 0.10 mL of sample 2 of Part A was pipetted into sample 2 of Table 2, 0.10 mL of sample 3 of Part A was pipetted into sample 3 of Table 2, 0.10 mL of sample 4 of Part A was pipetted into sample 4 of Table 2, and 0.10 mL of sample 5 of Part A was pipetted into sample 5 of Table 2. Mixing were performed immediately by inversion, and the 253 nm increment within about 5 minutes was recorded. Using the maximum linear rate, the ΔA253nm/min values of the sample, the blank sample, and the non-inhibited group were obtained respectively.

**Table 3**

| | | | | | | | |
|---|---|---|---|---|---|---|---|
| Non-inhibited group (Part A) | 0.10 | - | - | - | - | - | - |
| Sample 1 (Part A) | - | 0.10 | - | - | - | - | - |
| Sample 2 (Part A) | - | - | 0.10 | - | - | - | - |
| Sample 3 (Part A) | - | - | - | 0.10 | - | - | - |
| Sample 4 (Part A) | - | - | - | - | 0.10 | - | - |
| Sample 5 (Part A) | - | - | - | - | - | 0.10 | - |

Calculation $Trypsin activity in BAEE units / mL enzyme = \frac{\left(ΔA253nm/min sample-ΔA253nm/min blank\right) \left(df\right) \left(10.0\right)}{\left(0.001\right) \left(0.10\right) \left(0.5\right)}$ df = dilution factor
0.001 = value of ΔA₂₅₃ produced per minute by consuming 1 unit of BAEE under conditions of a pH value of 7.6, a temperature of 25°C, and a reaction volume of 3.20 mL
0.10 = volume of enzyme used (in milliliters) (Part B)
10.0 = total volume in milliliters (Part A)
0.5 = volume of trypsin solution used (in milliliters) (Part A) $BAEE units / mg solid = \frac{BAEE units / mL enzyme}{mg solid / mL enzyme}$

A plot of trypsin activity (BAEE units/mg trypsin) versus mg trypsin inhibitor/RM was drawn. mg trypsin inhibitor = (mL trypsin inhibitor) trypsin inhibitor concentration, mg/mL) $mg trypsin inhibited by 1 mg trypsin inhibitor = \frac{mg trypsin / RM \left(standardization factor\right)}{mg trypsin inhibitor}$ 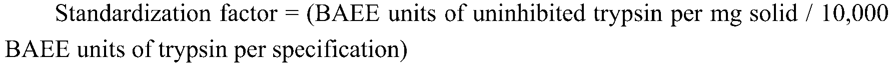 Standardization factor = (BAEE units of uninhibited trypsin per mg solid / 10,000 BAEE units of trypsin per specification)

### RM = reaction mixture

Chymotrypsin inhibitory activity was measured using chymotrypsin having a chymotrypsin activity of 40 BTEE units/mg, and expressed as mg of chymotrypsin inhibited per mg of test protein.

Principle: BTEE was used as a substrate, and the chymotrypsin inhibitory activity of soybean trypsin inhibitor was determined by measuring the absorbance at 256 nm of a reaction mixture containing the substrate (BAEE), chymotrypsin, and the soybean trypsin inhibitor at pH 7.8 and T=25°C.

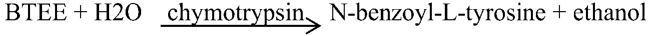

The name of BTEE is N-benzoyl-L-tyrosine ethyl ester

Unit definition:
Using BTEE as a reaction substrate, under conditions of a pH value of 7.8 and a temperature of 25°C, 1 unit of chymotrypsin activity will catalyze the hydrolysis reaction of 1 µmol of BTEE per minute.

Specifically:
The following reagents were formulated
a. 80 mM Tris-HCl buffer, pH 7.8, 25°C, which was buffer I
b. 50% (v/v) methanol, formulated using purified water and methanol
c. 1.18 mM BTEE (50 mL was formulated by dissolving BTEE in reagent b)
d. 2 M calcium chloride solution (5 mL was formulated by dissolving calcium chloride in ultrapure water)
e. 1 mM hydrochloric acid (50 milliliters was formulated by adding hydrochloric acid in ultrapure water)
f. 10 mM Tris-HCl buffer, pH 7.8, 25°C, which was buffer II (50 milliliters was formulated by adding hydrochloric acid in ultrapure water)
g. 2 mg/mL chymotrypsin solution, freshly formulated before use by using cold reagent e and chymotrypsin
h. 1 mg/mL chymotrypsin inhibitor solution (TCI): freshly formulated before use by using cold reagent f and the chymotrypsin inhibitor

Part A:
The following reagents were pipetted (in mL) into 6 centrifuge tubes respectively according to Table 4, followed by inversion and mixing.

**Table 4**

| | Non-inhibited group (mL) | Sample 1 (mL) | Sample 2 (mL) | Sample 3 (mL) | Sample 4 (mL) | Sample 5 (mL) |
|---|---|---|---|---|---|---|
| Reagent h (TCI solution) | - | 0.05 | 0.075 | 0.10 | 0.15 | 0.20 |
| Reagent g (chymotrypsin solution) | 0.50 | 0.50 | 0.50 | 0.50 | 0.50 | 0.50 |
| Reagent f (buffer II) | 9.50 | 9.45 | 9.425 | 9.40 | 9.35 | 9.30 |

Part B:
The following reagents were pipetted (in mL) into the 7 centrifuge tubes respectively according to Table 5 below, followed by inversion and mixing.

**Table 5**

| | Non-inhibited group (mL) | Sample 1 (mL) | Sample 2 (mL) | Sample 3 (mL) | Sample 4 (mL) | Sample 5 (mL) | Blank |
|---|---|---|---|---|---|---|---|
| Reagent c (BTEE) | 1.40 | 1.40 | 1.40 | 1.40 | 1.40 | 1.40 | 1.40 |
| Reagent a (buffer I) | 1.42 | 1.42 | 1.42 | 1.42 | 1.42 | 1.42 | - |
| Reagent d (calcium chloride) | 0.08 | 0.08 | 0.08 | 0.08 | 0.08 | 0.08 | 0.08 |
| Reagent f (buffer II) | - | - | - | - | - | 0.10 | - |

Each centrifuge tube was inverted to mix the content and equilibrated to 25°C. A256nm was monitored using a UV-visible spectrophotometer until constant. Reagents of Table 6 were added to the corresponding mixture of Table 5, specifically, 0.10 mL of the non-inhibited group of Part A in Table 6 was pipetted into the non-inhibited group of Table 5, 0.10 mL of sample 1 of Part A in Table 6 was pipetted into sample 1 of Table 5, 0.10 mL of sample 2 of Part A in Table 6 was pipetted into sample 2 of Table 5, 0.10 mL of sample 3 of Part A in Table 6 was pipetted into sample 3 of Table 5, 0.10 mL of sample 4 of Part A in Table 6 was pipetted into sample 4 of Table 5, and 0.10 mL of sample 5 of Part A in Table 6 was pipetted into sample 5 of Table 5. Inversion and mixing were performed immediately, and the 256 nm increment within about 5 minutes was recorded. Using the maximum linear rate, the ΔA256nm/min values of the sample solution, the blank sample solution, and the non-inhibited group solution were obtained respectively.

**Table 6**

| | | | | | | | |
|---|---|---|---|---|---|---|---|
| Non-inhibited group (Part A) | 0.10 | - | - | - | - | - | - |
| Sample 1 (Part A) | - | 0.10 | - | - | - | - | - |
| Sample 2 (Part A) | - | - | 0.10 | - | - | - | - |
| Sample 3 (Part A) | - | - | - | 0.10 | - | - | - |
| Sample 4 (Part A) | - | - | - | - | 0.10 | - | - |
| Sample 5 (Part A) | - | - | - | - | - | 0.10 | - |

Calculation $BTEE units / mL = \frac{\left(Δ\frac{A 256 nm}{min}Sample-Δ\frac{A 256 nm}{min}Blank group\right) \left(3\right) \left(0.5\right) \left(df\right)}{\left(0.964\right) \left(0.1\right) \left(10\right)}$
3 = total volume of Part B (mL)
0.5 = volume of chymotrypsin solution of Part A (mL)
df = dilution factor
0.964 = molar extinction coefficient of BAEE at 256nm;
0.1 = volume of chymotrypsin solution of Part A pipetted (mL), used in Part B
10 = total volume of Part A (mL)

A plot of chymotrypsin activity (BTEE units/mL) versus trypsin volume (mL) was drawn, the intersection point of the straight line in the plot with the abscissa was determined, thereby obtaining an X-axis intercept.

Y₀ = mg of chymotrypsin inhibitor that leads to complete inhibition of chymotrypsin activity per mL of chymotrypsin inhibitor solution in Part B of the experiment;

CT = mg of chymotrypsin per milliliter of chymotrypsin solution in Part B of the experiment; $\frac{CT}{Y 0} = mg$ of chymotrypsin inhibited by 1 mg of trypsin inhibitor.

For example: the TI activity of KTI was 1.4 mg/mg, indicating that each mg of KTI could inhibit 1.4 mg of trypsin, and the trypsin had a trypsin activity of 10,000 BAEE units/mg.

The CI activity of BBI was 1.5 mg/mg, indicating that each mg of BBI could inhibit 1.5 mg of chymotrypsin, and the chymotrypsin had a chymotrypsin activity of 40 BTEE units/mg.

The BBI prepared in Example 1 was tested, and the results were as shown in Table 7 below:

**Table 7**

| BBI Test Item | Test Result |
|---|---|
| Appearance | White |
| Trypsin inhibitory activity (TI) | 1 mg of BBI inhibits 0.9 mg of trypsin with a trypsin activity of approximately 10,000 BAEE units/mg |
| Chymotrypsin inhibitory activity (CI) | 1 mg of BBI inhibits 1.5 mg of chymotrypsin with a chymotrypsin activity of approximately 40 BTEE units/mg |
| BBI content determined by BCA method | 92.0% |
| Purity determined by SDS-PAGE and | Purity: 91.0%; high-molecular-weight |
| imaging quantitation | impurities (>30,000): <0.1% |
| Moisture content | 9.1% |

The KTI prepared in Example 1 was tested, and the results were as shown in Table 8 below:

**Table 8**

| KTI Test Item | Test Result |
|---|---|
| Appearance | White |
| Trypsin inhibitory activity (TI) | 1 mg of KTI inhibits 1.4 mg of trypsin with a trypsin activity of approximately 10,000 BAEE units/mg |
| KTI content determined by BCA method | 90.0% |
| Purity determined by SDS-PAGE and imaging quantitation | Purity: 100%; high-molecular-weight impurities (>30,000): <0.1% |
| Moisture content | 8.0% |

In the following examples, unless otherwise specified, the reagents and consumables used were purchased from conventional reagent manufacturers in the art; unless otherwise specified, the experimental methods and technical means used were conventional methods and means in the art.

For example, recombinant human insulin could be purchased from Hefei Tianmai Biotechnology Development Co., Ltd., fish oil meeting the "Omega-3-acid triglycerides" quality standard of the European Pharmacopoeia (EP 10.8) could be procured, disodium ethylenediaminetetraacetate could be purchased from Shanghai Aladdin Biochemical Technology Co., Ltd., and Tween 80 could be purchased from Sigma-Aldrich.

### Example 3 Preparation of Oral Polypeptide Composition

This example provides an oral polypeptide composition, wherein a mass ratio of recombinant human insulin, fish oil, soybean trypsin inhibitor, disodium ethylenediaminetetraacetate, and Tween 80 contained in the oral polypeptide composition was 1:40:20:15:40, wherein the mass of the recombinant human insulin was 8 mg.

Preparation method: 8 mg of recombinant human insulin, 320 mg of fish oil, 160 mg of soybean trypsin inhibitor, 120 mg of disodium ethylenediaminetetraacetate, and 320 mg of Tween 80 were weighed separately; wherein the 160 mg of soybean trypsin inhibitor was obtained by mixing 26.7 mg of BBI and 133.3 mg of KTI obtained in Example 1. Then, the above weighed recombinant human insulin, BBI, KTI, and disodium ethylenediaminetetraacetate were mixed, and the weighed fish oil and Tween 80 were added thereto, followed by mixing uniformly and loading into a gelatin capsule. The capsule was coated, wherein the coating materials included 40 mg of Eudragit L-100, 30 mg of talc, and 5 mg of polyethylene glycol 6000. The above coating materials were dissolved in an appropriate amount of dichloromethane and isopropanol to obtain a coating solution, and the coating solution was sprayed onto the surface of the capsule to obtain the oral polypeptide composition.

Regarding the oral polypeptide composition prepared in this example, the capsule contents included recombinant human insulin, fish oil, BBI, KTI, disodium ethylenediaminetetraacetate, and Tween 80. The capsule contents were a suspension and were anhydrous. "Anhydrous" meant that in the process of preparing the oral polypeptide composition of this example, water was not intentionally added to the capsule contents, but it did not exclude that each component in the capsule contents absorbed water from the air.

Through calculation, in the oral polypeptide composition obtained in this example, the mass fraction of recombinant human insulin was 0.9%, the mass fraction of fish oil was 34.5%, the mass fraction of soybean trypsin inhibitor was 17.2%, the mass fraction of disodium ethylenediaminetetraacetate was 12.9%, and the mass fraction of Tween 80 was 34.5%. The mass fraction of BBI was 2.9%, and the mass fraction of KTI was 14.3%.

The weightratio of BBI:KTI in each capsule was 0.2:1. The trypsin inhibitory activity of KTI used in the capsule was 1.4 mg/mg. The trypsin inhibitory activity of BBI used in the capsule was 0.9 mg/mg, and the chymotrypsin inhibitory activity of BBI was 1.5 mg/mg. The total TI/total CI activity ratio in each capsule was 5.27:1.

### Example 4 Preparation of Oral Polypeptide Composition

This example provides an oral polypeptide composition, wherein the oral polypeptide composition contains recombinant human insulin, fish oil, soybean trypsin inhibitor, disodium ethylenediaminetetraacetate, and Tween 80, wherein the mass of the recombinant human insulin was 16 mg.

Preparation method: 16 mg of recombinant human insulin, 105 mg of fish oil, 200 mg of soybean trypsin inhibitor, 320 mg of disodium ethylenediaminetetraacetate, and 245 mg of Tween 80 were weighed separately; wherein the 200 mg of soybean trypsin inhibitor was obtained by mixing 88.9 mg of BBI and 111.1 mg of KTI obtained in Example 1. Then, the above weighed recombinant human insulin, BBI, KTI, and disodium ethylenediaminetetraacetate were mixed, and the weighed fish oil and Tween 80 were added thereto, followed by mixing uniformly and loading into a gelatin capsule. The capsule was coated, wherein the coating materials included 40 mg of Eudragit L-100, 30 mg of talc, and 5 mg of polyethylene glycol 6000. The above coating materials were dissolved in an appropriate amount of dichloromethane and isopropanol to obtain a coating solution, and the coating solution was sprayed onto the surface of the capsule to obtain the oral polypeptide composition.

Regarding the oral polypeptide composition prepared in this example, the capsule contents included recombinant human insulin, fish oil, BBI, KTI, disodium ethylenediaminetetraacetate, and Tween 80. The capsule contents were a suspension and were anhydrous. "Anhydrous" meant that in the process of preparing the oral polypeptide composition of this example, water was not intentionally added to the capsule contents, but it did not exclude that each component in the capsule contents absorbed water from the air.

Through calculation, in the oral polypeptide composition obtained in this example, the mass fraction of recombinant human insulin was 1.8%, the mass fraction of fish oil was 11.9%, the mass fraction of soybean trypsin inhibitor was 22.6%, the mass fraction of disodium ethylenediaminetetraacetate was 36.1%, and the mass fraction of Tween 80 was 27.6%. The mass fraction of BBI was 10.0%, and the mass fraction of KTI was 12.6%.

The weight ratio of BBI:KTI in each capsule was 0.8:1. The trypsin inhibitory activity of KTI used in the capsule was 1.4 mg/mg. The trypsin inhibitory activity of BBI used in the capsule was 0.9 mg/mg, and the chymotrypsin inhibitory activity of BBI was 1.5 mg/mg. The total TI/total CI activity ratio in each capsule was 1.77:1.

### Example 5 Preparation of Oral Polypeptide Composition

This example provides an oral polypeptide composition, wherein the oral polypeptide composition contains recombinant human insulin, fish oil, soybean trypsin inhibitor, disodium ethylenediaminetetraacetate, and Tween 80, wherein the mass of the recombinant human insulin was 32 mg.

Preparation method: 32 mg of recombinant human insulin, 245 mg of fish oil, 70 mg of soybean trypsin inhibitor, 250 mg of disodium ethylenediaminetetraacetate, and 105 mg of Tween 80 were weighed separately; wherein the 70 mg of soybean trypsin inhibitor was obtained by mixing 52.5 mg of BBI and 17.5 mg of KTI obtained in Example 1. Then, the above weighed recombinant human insulin, BBI, KTI, and disodium ethylenediaminetetraacetate were mixed, and the weighed fish oil and Tween 80 were added thereto, followed by mixing uniformly and loading into a gelatin capsule. The capsule was coated, wherein the coating materials included 40 mg of Eudragit L-100, 30 mg of talc, and 5 mg of polyethylene glycol 6000. The above coating materials were dissolved in an appropriate amount of dichloromethane and isopropanol to obtain a coating solution, and the coating solution was sprayed onto the surface of the capsule to obtain the oral polypeptide composition.

Regarding the oral polypeptide composition prepared in this example, the capsule contents included recombinant human insulin, fish oil, BBI, KTI, disodium ethylenediaminetetraacetate, and Tween 80. The capsule contents were a suspension and were anhydrous. "Anhydrous" meant that in the process of preparing the oral polypeptide composition of this example, water was not intentionally added to the capsule contents, but it did not exclude that each component in the capsule contents absorbed water from the air.

Through calculation, in the oral polypeptide composition obtained in this example, the mass fraction of recombinant human insulin was 4.6%, the mass fraction of fish oil was 34.9%, the mass fraction of soybean trypsin inhibitor was 10.0%, the mass fraction of disodium ethylenediaminetetraacetate was 35.6%, and the mass fraction of Tween 80 was 14.9%. The mass fraction of BBI was 7.5%, and the mass fraction of KTI was 2.5%.

The weight ratio of BBI:KTI in each capsule was 3:1. The trypsin inhibitory activity of KTI used in the capsule was 1.4 mg/mg. The trypsin inhibitory activity of BBI used in the capsule was 0.9 mg/mg, and the chymotrypsin inhibitory activity of BBI was 1.5 mg/mg. The total TI/total CI activity ratio in each capsule was 0.91:1.

### Example 6 Preparation of Oral Polypeptide Composition

This example provides an oral polypeptide composition, wherein the oral polypeptide composition contains recombinant human insulin, fish oil, soybean trypsin inhibitor, disodium ethylenediaminetetraacetate, and Tween 80, wherein the mass of the recombinant human insulin was 8 mg.

Preparation method: 8 mg of recombinant human insulin, 50 mg of fish oil, 100 mg of soybean trypsin inhibitor, 300 mg of disodium ethylenediaminetetraacetate, and 450 mg of Tween 80 were weighed separately; wherein the 100 mg of soybean trypsin inhibitor was obtained by mixing 37.5 mg of BBI and 62.5 mg of KTI obtained in Example 1. Then, the above weighed recombinant human insulin, BBI, KTI, and disodium ethylenediaminetetraacetate were mixed, and the weighed fish oil and Tween 80 were added thereto, followed by mixing uniformly and loading into a gelatin capsule. The capsule was coated, wherein the coating materials included 40 mg of Eudragit L-100, 30 mg of talc, and 5 mg of polyethylene glycol 6000. The above coating materials were dissolved in an appropriate amount of dichloromethane and isopropanol to obtain a coating solution, and the coating solution was sprayed onto the surface of the capsule to obtain the oral polypeptide composition.

Regarding the oral polypeptide composition prepared in this example, the capsule contents included recombinant human insulin, fish oil, BBI, KTI, disodium ethylenediaminetetraacetate, and Tween 80. The capsule contents were a suspension and were anhydrous. "Anhydrous" meant that in the process of preparing the oral polypeptide composition of this example, water was not intentionally added to the capsule contents, but it did not exclude that each component in the capsule contents absorbed water from the air.

Through calculation, in the oral polypeptide composition obtained in this example, the mass fraction of recombinant human insulin was 0.9%, the mass fraction of fish oil was 5.5%, the mass fraction of soybean trypsin inhibitor was 11.0%, the mass fraction of disodium ethylenediaminetetraacetate was 33.0%, and the mass fraction of Tween 80 was 49.6%. The mass fraction of BBI was 4.0%, and the mass fraction of KTI was 7.0%.

The weight ratio of BBI:KTI in each capsule was 0.6:1. The trypsin inhibitory activity of KTI used in the capsule was 1.4 mg/mg. The trypsin inhibitory activity of BBI used in the capsule was 0.9 mg/mg, and the chymotrypsin inhibitory activity of BBI was 1.5 mg/mg. The total TI/total CI activity ratio in each capsule was 2.16:1.

### Example 7 - Comparative Example

This example provides an oral polypeptide composition, wherein the oral polypeptide composition contains recombinant human insulin, soybean trypsin inhibitor, disodium ethylenediaminetetraacetate, and Tween 80, wherein the mass of the recombinant human insulin was 16 mg.

Preparation method: 16 mg of recombinant human insulin, 200 mg of soybean trypsin inhibitor, 320 mg of disodium ethylenediaminetetraacetate, and 350 mg of Tween 80 were weighed separately; wherein the 200 mg of soybean trypsin inhibitor was obtained by mixing 88.9 mg of BBI and 111.1 mg of KTI obtained in Example 1. Then, the above weighed recombinant human insulin, BBI, KTI, and disodium ethylenediaminetetraacetate were mixed, and the weighed Tween 80 was added thereto, followed by mixing uniformly and loading into a gelatin capsule. The capsule was coated, wherein the coating materials included 40 mg of Eudragit L-100, 30 mg of talc, and 5 mg of polyethylene glycol 6000. The above coating materials were dissolved in an appropriate amount of dichloromethane and isopropanol to obtain a coating solution, and the coating solution was sprayed onto the surface of the capsule to obtain the oral polypeptide composition.

### Example 8 - Comparative Example

This example provides an oral polypeptide composition, wherein the oral polypeptide composition contains recombinant human insulin, fish oil, soybean trypsin inhibitor, and disodium ethylenediaminetetraacetate, wherein the mass of the recombinant human insulin was 16 mg.

Preparation method: 16 mg of recombinant human insulin, 350 mg of fish oil, 200 mg of soybean trypsin inhibitor, and 320 mg of disodium ethylenediaminetetraacetate were weighed separately; wherein the 200 mg of soybean trypsin inhibitor was obtained by mixing 88.9 mg of BBI and 111.1 mg of KTI obtained in Example 1. Then, the above weighed recombinant human insulin, BBI, KTI, and disodium ethylenediaminetetraacetate were mixed, and the weighed fish oil was added thereto, followed by mixing uniformly and loading into a gelatin capsule. The capsule was coated, wherein the coating materials included 40 mg of Eudragit L-100, 30 mg of talc, and 5 mg of polyethylene glycol 6000. The above coating materials were dissolved in an appropriate amount of dichloromethane and isopropanol to obtain a coating solution, and the coating solution was sprayed onto the surface of the capsule to obtain the oral polypeptide composition.

### Example 9 Stability Test

The recombinant human insulin content in the oral polypeptide compositions of Example 3, Example 4, Example 5, and Example 6 was determined by HPLC after being placed at 25°C for 0 days, 5 days, 10 days, 30 days, and 60 days, respectively. Three samples of each oral polypeptide composition were determined at each time point, and the obtained results were as shown in Table 9. The content in Table 9 was an average value of the three samples at each time point.

**Table 9. Stability data of recombinant human insulin at 25°C**

| Oral polypeptide composition | Recombinant human insulin content (%) | | | | |
|---|---|---|---|---|---|
| | Day 0 | Day 5 | Day 10 | Day 30 | Day 60 |
| Example 3 | 101.3% | 101.6% | 97.3% | 88.2% | 70.1% |
| Example 4 | 100.2% | 99.8% | 96.9% | 86.4% | 69.2% |
| Example 5 | 100.6% | 100.9% | 97.2% | 87.5% | 63.4% |
| Example 6 | 99.6% | 98.3% | 96.2% | 85.4% | 62.2% |

"Recombinant human insulin content" refers to the percentage of an actual content of recombinant human insulin to a labeled amount. For example, the labeled amount of recombinant human insulin in an oral polypeptide composition was 8 mg; after a period of storage, 3 samples were determined by HPLC, and the actual content of recombinant human insulin (average value) was calculated as 4.8 mg. Then the recombinant human insulin content in the oral polypeptide composition was $\frac{4.8 mg}{8.0 mg} \times 100 % = 60 %$.

HPLC conditions for recombinant human insulin content:
Chromatographic column: octadecylsilane bonded silica gel;
Mobile phase A: 600 mL of 0.1 M sodium sulfate buffer (pH 4.8), 100 mL of acetonitrile, and 300 mL of water;
Mobile phase B: 100 mL of 0.1 M sodium sulfate buffer (pH 4.8), 600 mL of acetonitrile, and 300 mL of water;
Flow rate: 1.0 mL/min; Column temperature: 35°C; Detection wavelength: UV 214 nm; Injection volume: 20 µL.

A gradient elution schedule was as follows:

**Table 10 Gradient elution schedule**

| Time (min) | Mobile phase A (%) | Mobile phase B (%) |
|---|---|---|
| 0 | 80 | 20 |
| 15 | 80 | 20 |
| 16 | 0 | 100 |
| 21 | 0 | 100 |
| 22 | 90 | 10 |
| 30 | 90 | 10 |

It could be seen from Table 9 that, when the oral polypeptide composition was placed at 25°C for 30 days, the recombinant human insulin content was greater than 85%; when placed for 60 days, the recombinant human insulin content was greater than 62%.

The recombinant human insulin content in the oral polypeptide compositions of Example 3, Example 4, Example 5, and Example 6 was determined by HPLC after being placed at 40°C for 0 days, 5 days, and 10 days, respectively. Three samples of each oral polypeptide composition were determined at each time point, and the obtained results were as shown in Table 11. The content in Table 11 was an average value of the three samples at each time point.

**Table 11. Stability data of recombinant human insulin at 40°C**

| Oral polypeptide composition | Recombinant human insulin content (%) | | |
|---|---|---|---|
| | Day 0 | Day 5 | Day 10 |
| Example 3 | 101.3% | 87.4% | 76.8% |
| Example 4 | 100.2% | 85.4% | 78.2% |
| Example 5 | 100.6% | 88.3% | 76.4% |
| Example 6 | 99.6% | 87.9% | 80.1% |

It could be seen from Table 11 that, when the oral polypeptide composition was placed at 40°C for 5 days, the recombinant human insulin content was greater than 85%; when placed for 10 days, the recombinant human insulin content was greater than 76%.

### Example 10

### Dissolution Test

According to the dissolution determination method specified in Method 2 of General Rule 0931, Volume IV of the Chinese Pharmacopoeia 2020 Edition, a phosphate buffer with a pH of 6.8 was used as a dissolution medium. The contents of the oral polypeptide compositions prepared in Example 3, Example 4, Example 5, Example 6, Example 7, and Example 8 were poured into different dissolution vessels, respectively, and dissolved in the above dissolution medium. The rotational speed was 50 revolutions per minute, the temperature of the dissolution medium was constant at 37°C ± 0.5°C for dissolution test. Samples were taken at 0 min, 2 min, 5 min, 10 min, 20 min, 30 min, 45 min, and 60 min, and the recombinant human insulin content was determined by HPLC. Cumulative dissolution data were as shown in Table 12.

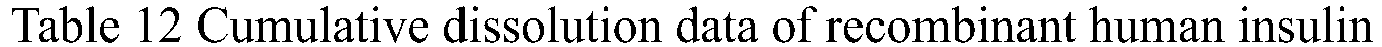

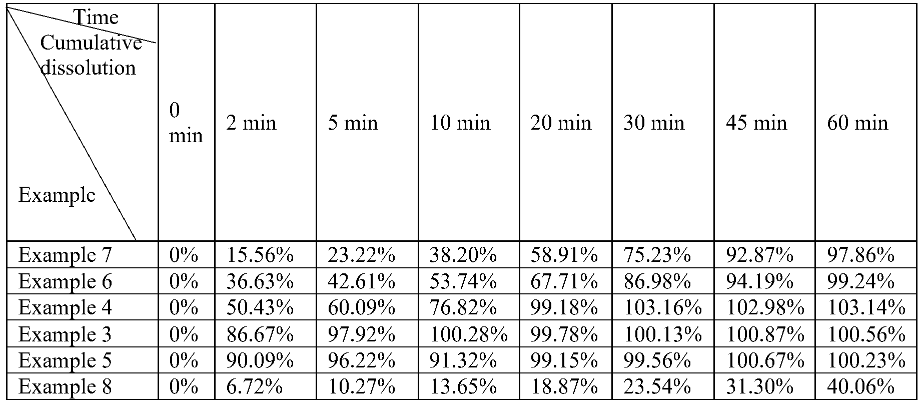

HPLC conditions for recombinant human insulin content:
Chromatographic column: octadecylsilane bonded silica gel;
Mobile phase A: 600 mL of 0.1 M sodium sulfate buffer (pH 4.8), 100 mL of acetonitrile, and 300 mL of water;
Mobile phase B: 100 mL of 0.1 M sodium sulfate buffer (pH 4.8), 600 mL of acetonitrile, and 300 mL of water;
Flow rate: 1.0 mL/min; Column temperature: 35°C; Detection wavelength: UV 214 nm; Injection volume: 20 µL.

A gradient elution schedule was as follows:

**Table 13 Gradient elution schedule**

| Time (min) | Mobile phase A (%) | Mobile phase B (%) |
|---|---|---|
| 0 | 80 | 20 |
| 15 | 80 | 20 |
| 16 | 0 | 100 |
| 21 | 0 | 100 |
| 22 | 90 | 10 |
| 30 | 90 | 10 |

As could be seen from Table 12,
for the oral polypeptide composition of Example 6, the cumulative dissolution of recombinant human insulin at 30 min was greater than 86%, and the dissolution was basically complete at 45 min.

For the oral polypeptide composition of Example 4, the dissolution of recombinant human insulin was basically complete at 20 min.

For the oral polypeptide compositions of Example 3 and Example 5, the cumulative dissolution of recombinant human insulin at 2 min was greater than 86%, and the dissolution was basically complete at 5 min.

### Dissolution test

According to the dissolution determination method specified in Method 2 of General Rule 0931, Volume IV of the Chinese Pharmacopoeia 2020 Edition, a phosphate buffer with a pH of 6.8 was used as a dissolution medium. The contents of the oral polypeptide compositions prepared in Example 3, Example 4, Example 5, and Example 6 were poured into different dissolution vessels, respectively, and dissolved in the above dissolution medium. The rotational speed was 50 revolutions per minute, the temperature of the dissolution medium was constant at 37°C ± 0.5°C for the dissolution test. Samples were taken at 0 min, 2 min, 5 min, 10 min, 20 min, 30 min, 45 min, and 60 min, and the soybean trypsin inhibitor content was determined by HPLC. Cumulative dissolution data were as shown in Table 14.

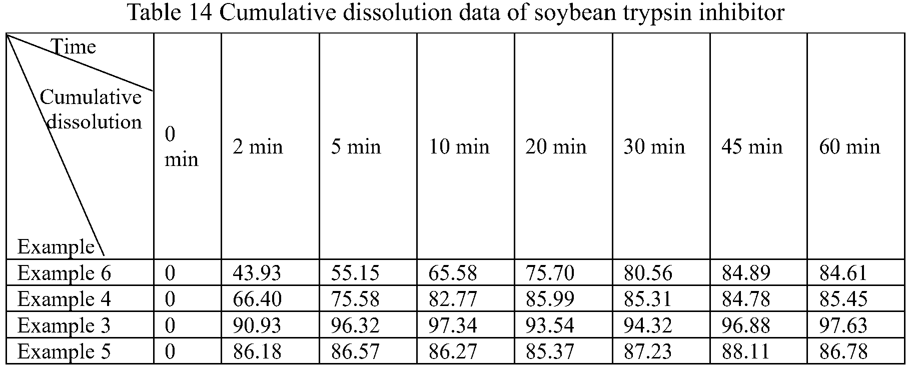

HPLC conditions for soybean trypsin inhibitor content:
Mobile phase A: water-acetonitrile-trifluoroacetic acid (95 mL: 5 mL: 0.05 mL);
Mobile phase B: water-acetonitrile-trifluoroacetic acid (10 mL: 90 mL: 0.05 mL);
Chromatographic column: C18;
Flow rate: 1 mL/min;
Column temperature: 40°C;
Detector: UV 214 nm;
Injection volume: 20 µL;

A gradient elution schedule was as follows:

**Table 15 Gradient elution schedule**

| No. | Time (min) | Mobile phase A (%) | Mobile phase B (%) |
|---|---|---|---|
| 1 | 0 | 100 | 0 |
| 2 | 15 | 40 | 60 |
| 4 | 15.01 | 100 | 0 |
| 5 | 25 | 100 | 0 |
| 8 | 25.01 | 100 | 0 |
| 9 | 35 | 100 | 0 |

As could be seen from Table 14, in the oral polypeptide compositions of Example 3 and Example 5, the dissolution of the soybean trypsin inhibitor was faster than that in the oral polypeptide compositions of Example 4 and Example 6. In the oral polypeptide composition of Example 3, the dissolution of the soybean trypsin inhibitor was basically complete at 5 min. The dissolution of the soybean trypsin inhibitor in the oral polypeptide composition of Example 4 was faster than the dissolution of the soybean trypsin inhibitor in the oral polypeptide composition of Example 6.

### Example 11

### Animal Test

An animal test was performed by using the oral polypeptide compositions prepared in Examples 3 to 6.

### (1) Experimental animals

Beagle dogs, male, 8 to 10 kg.

### (2) Animal grouping and administration design

Group design: 4 groups were set up for the test, i.e., low-dose group 1, medium-dose group, high-dose group, and low-dose group 2, respectively;

Animal quantity: A total of 8 animals in each administration group, male;

Test method: A total of 2 rounds of tests were performed, with 4 groups in each round and 4 animals in each group; a washout period was set to 48 h;

Grouping method: Random grouping was performed according to body weight. Specific grouping information was as shown in Table 16.

**Table 16 Dose and Grouping Information Table**

| Group | | Vehicle/ Test Substanc e | Dose | Administr ation quantity | Administr ation Route | Administr ation Frequency | Numb er of Anim als | Animal ID [1] |
|---|---|---|---|---|---|---|---|---|
| | Round 1 Test | | | | | | | |
| 1 | Low-dose group 1 | Oral polypepti de composit ion of Example 3 | 8 mg /animal | 1 capsule | Oral | Single dose | 4 | 1M001-1 M004 |
| 2 | Medium-d ose group | Oral polypepti de composit ion of Example 4 | 16 mg/ani mal | 1 capsule | Oral | Single dose | 4 | 2M001-2 M004 |
| 3 | High-dose group | Oral polypepti de composit ion of Example 5 | 32 mg/ani mal | 1 capsule | Oral | Single dose | 4 | 3M001-3 M004 |
| 4 | Low-dose group 2 | Oral polypepti de composit ion of Example 6 | 8 mg/ani mal | 1 capsule | Oral | Single dose | 4 | 4M001-4 M004 |

| Round 2 Test | | | | | | | | |
|---|---|---|---|---|---|---|---|---|
| 1 | Low-dose group 1 | Oral polypepti de composit ion of Example 3 | 8 mg /animal | 1 capsule | Oral | Single dose | 4 | 1M005-1 M008 |
| 2 | Medium-dose group | Oral polypepti de composit ion of Example 4 | 16 mg/ani mal | 1 capsule | Oral | Single dose | 4 | 2M005-2 M008 |
| 3 | High-dos e group | Oral polypepti de composit ion of Example 5 | 32 mg/ani mal | 1 capsule | Oral | Single dose | 4 | 3M005-3 M008 |
| 4 | Low-dose group 2 | Oral polypepti de composit ion of Example 6 | 8 mg/ani mal | 1 capsule | Oral | Single dose | 4 | 4M005-4 M008 |

| | | | | | | | | |
|---|---|---|---|---|---|---|---|---|
| Notes: [1] The first digit of the animal number represented the group, the second letter represented the gender (M for male), and the 3^{rd}, 4^{th}, and 5^{th} digits represented the individual animal number; [2] After the Round 1 Test ended, after a washout of 48 h, random grouping was performed, and then the Round 2 Test was performed; [3] Fasting overnight for more than 12 h was performed prior to administration. | | | | | | | | |

### (3) Administration method

Low-dose group 1, medium-dose group, high-dose group, and low-dose group 2
Administration route: oral administration;
Administration frequency: administered once;
Administration quantity: 1 capsule.

### (4) Detection method

Blood collection time: blood was collected from a forelimb vein using a vacuum blood collection tube (heparin anticoagulation) at time points of 0 (before administration), 0.25, 0.5, 0.75, 1.0, 1.5, 2.0, 2.5, 3.0, 4.0, 6.0, and 8.0 h, with a blood collection volume of about 2.5 mL each time. After whole blood collection, it was placed at a low temperature, and centrifuged at 4°C (3000 rpm × 15 min) within 2 h to separate plasma. All samples to be tested were stored at -70°C until determination. A human insulin concentration in plasma was detected by LC/MS/MS.

**Table 17 LC/MS/MS detection conditions**

| **Item** | **Parameter** |
|---|---|
| Ion source | Electrospray |
| Ionization mode | Positive ion mode |
| Scan mode | Multiple Reaction Monitoring (MRM) |
| Internal standard | Insulin glargine |
| Column | BEH C4 1.7 µm 300 Å (50 mm * 2.10 mm) Column |
| Mobile phase | A = 1% trifluoroacetic acid - H₂O; B = 1% trifluoroacetic acid - acetonitrile |
| Autosampler temperature | 15°C |
| Column temperature | 40°C |
| Injection volume | 10 µL |
| Flow rate | 0.5 mL/min |
| Gradient elution | 0.40 min, B was 20.0%; 2.40 min, B was 40.0%; 2.50 min, B was 95.0%; 3.00 min, B was 95.0%; 3.01 min, B was 20.0%; 3.50 min, B was 20.0%; 4.00 min, B was 95.0%; 4.50 min, B was 95.0%; 4.51 min, B was 20.0%; 5.00 min, B was 20.0% |

Conclusion:
Calculation results of the absolute bioavailability F of the oral administration groups were as shown in Table 18 below:

**Table 18. Absolute bioavailability F for oral administration**

| Test group | F (%) (Mean) (SD) |
|---|---|
| Low-dose group 1 | 1.54 (0.43) |
| Medium-dose group | 1.61 (0.45) |
| High-dose group | 1.59 (0.32) |
| Low-dose group 2 | 1.38 (0.24) |

As could be seen from Table 18, the absolute bioavailability of low-dose group 1 was 1.54%, and the SD (standard deviation) was 0.43.

As a comparison, when the same test scheme was adopted for the oral polypeptide composition in which the soybean trypsin inhibitor was replaced with a commercially available soybean trypsin inhibitor directly extracted from soybeans (rather than separately obtaining BBI and KTI from soybeans followed by mixing them to obtain the soybean trypsin inhibitor and the rest remained unchanged, the F of each comparative example was as shown in Table 19 below:

**Table 19. Absolute bioavailability (F) of comparative examples**

| Comparative experimental group | F (%) (Mean) (SD) |
|---|---|
| Comparative low-dose group 1 | 1.68 (0.84) |
| Comparative medium-dose group | 1.46 (0.70) |
| Comparative high-dose group | 1.31 (0.93) |
| Comparative low-dose group 2 | 1.52 (0.87) |

As could be seen from Table 19, the absolute bioavailability of comparative low-dose group 1 was 1.68%, and the SD (standard deviation) was 0.84.

The applicant states that the above descriptions are only preferred embodiments of the present invention, and are only used to illustrate the technical solutions of the present invention, rather than to limit the protection scope of the present invention. Any modifications, equivalent replacements, or improvements, etc. made within the spirit and principles of the present invention shall all be included within the protection scope of the present invention.

## Claims

1. A polypeptide composition comprising a polypeptide, an isolated BBI and an isolated KTI, fish oil, and at least one absorption enhancer; wherein the isolated BBI and the isolated KTI are mixed in a certain ratio, so that an activity ratio of a total trypsin inhibitory activity (total TI) to a total chymotrypsin inhibitory activity (total CI) of the composition is from 0.5:1 to 10:1; preferably from 0.7:1 to 10:1; preferably from 0.91:1 to 6:1; preferably from 0.91:1 to 5.27:1.

2. The polypeptide composition according to claim 1, wherein the isolated BBI has been purified to a purity of at least 85% as measured by sodium dodecyl sulfate polyacrylamide gel electrophoresis (SDS-PAGE); preferably, the BBI has been purified to a purity of at least 90%; more preferably, the BBI has been purified to a purity of at least 91%.

3. The polypeptide composition according to claim 1 or 2, wherein the isolated BBI has been purified to a protein content of at least 85% as measured by a BCA (Bicinchoninic acid) assay; preferably, the BBI has been purified to a protein content of at least 90%; more preferably, the BBI has been purified to a protein content of at least 92%.

4. The polypeptide composition according to any one of claims 1 to 3, wherein the isolated BBI contains less than 0.1% high molecular weight impurities.

5. The polypeptide composition according to any one of claims 1 to 4, wherein the isolated BBI has a trypsin inhibitory activity of at least 0.5, 0.6, 0.7, 0.8, 0.9 mg/mg, or 1.8 mg/mg; and the isolated BBI has a chymotrypsin inhibitory activity of at least 0.5, 0.6, 0.7, 0.8, 0.9, 1.0, 1.1, 1.2, 1.3, 1.4, 1.5 mg/mg, or 1.8 mg/mg.

6. The polypeptide composition according to any one of claims 1 to 5, wherein the isolated KTI has been purified to a purity of at least 85% as measured by SDS-PAGE; preferably, the isolated KTI has been purified to a purity of at least 90%; more preferably, the isolated KTI has been purified to a purity of at least 95%.

7. The polypeptide composition according to any one of claims 1 to 6, wherein the isolated KTI has been purified to a protein content of greater than 85% as measured by the BCA assay; preferably, the isolated KTI has been purified to a protein content of greater than 90%.

8. The polypeptide composition according to any one of claims 1 to 7, wherein the isolated KTI contains less than 0.1% high molecular weight impurities.

9. The polypeptide composition according to any one of claims 1 to 8, wherein the isolated KTI has a trypsin inhibitory activity of at least 0.5, 0.6, 0.7, 0.8, 0.9, 1.0, 1.1, 1.2, 1.3, 1.4 mg/mg, or 1.8 mg/mg.

10. The polypeptide composition according to any one of claims 1 to 9, wherein the polypeptide composition has a chymotrypsin inhibitory activity that inhibits 0.04 to 0.30 mg chymotrypsin/mg polypeptide composition, wherein the chymotrypsin has a chymotrypsin activity of 40 BTEE units/mg; preferably, wherein the polypeptide composition has a chymotrypsin inhibitory activity that inhibits 0.04 to 0.15 mg chymotrypsin/mg polypeptide composition, wherein the chymotrypsin has a chymotrypsin activity of 40 BTEE units/mg.

11. The polypeptide composition according to any one of claims 1 to 10, wherein the polypeptide composition has a trypsin inhibitory activity that inhibits 0.05 to 0.40 mg trypsin/mg polypeptide composition, wherein the trypsin has a trypsin activity of 10000 BAEE units/mg; preferably, wherein the polypeptide composition has a trypsin inhibitory activity that inhibits at least 0.10 to 0.30 mg trypsin/mg polypeptide composition, wherein the trypsin has a trypsin activity of 10000 BAEE units/mg.

12. The polypeptide composition according to any one of claims 1 to 11, wherein a molecular weight of the polypeptide is 100 Da to 20000 Da, preferably 1000 Da to 20000 Da, preferably 1000 Da to 6150 Da, preferably 1209 Da to 6104 Da, preferably 1000 Da to 6000 Da, and further preferably 2000 Da to 6000 Da.

13. The polypeptide composition according to any one of claims 1 to 12, wherein the polypeptide is selected from the group consisting of any one or more of insulin, glucagon, Glucagon-like peptide-1 receptor agonist, Glucagon-like peptide-2 receptor agonist, Calcitonin, growth hormone, somatostatin, thyrotropin-releasing hormone, parathyroid hormone, gonadotropin-releasing hormone, heparin, granulocyte colony-stimulating factor, prostaglandin, cyclosporine, interferon, vasopressin, vancomycin, erythropoietin, glutathione, and thymosin;
preferably, the polypeptide is selected from the group consisting of any one or more of recombinant human insulin, insulin degludec, glucagon, exenatide, benaglutide, liraglutide, loxenatide, dulaglutide, lixisenatide, semaglutide, albiglutide, dulaglutide, teduglutide, calcitonin, salmon calcitonin, growth hormone, octreotide, thyrotropin-releasing hormone, parathyroid hormone fragment, teriparatide, leuprorelin, protirelin, gonadorelin, goserelin, buserelin, sermorelin, nafarelin, histrelin, triptorelin, tesamorelin, corticorelin, lanreotide acetate, terlipressin, antide, teriparatide, abaloparatide, nesiritide, eptifibatide, lixisenatide, sifuvirtide, atosiban, trametes polysaccharopeptide, ginseng glycopeptide, bone peptide, carnosine and adenosine, glutathione, mannatide, enfuvirtide, bradykinin, enkephalin, nosiheptide, hirudin, glatiramer acetate, aprotinin, alarelin, pancreatic kallikrein, polymyxin, serrapeptase, thymosin α1, thymopentin, and thymalfasin.

14. The polypeptide composition according to any one of claims 1 to 13, wherein the absorption enhancer is selected from the group consisting of any one or more of ethylenediaminetetraacetic acid or a salt thereof, N-(8-(2-hydroxybenzoyl)amino)caprylic acid or a salt thereof, salicylic acid or a salt thereof, citric acid or a salt thereof, cholic acid or a salt thereof, deoxycholic acid or a salt thereof, glycocholic acid or a salt thereof, taurocholic acid or a salt thereof, alkyl glycoside, sodium dodecyl sulfate, docusate sodium, cyclodextrin or a derivative thereof, chitosan or a derivative thereof, caprylic acid or a salt thereof, capric acid or a salt thereof, lauric acid or a salt thereof, myristic acid, palmitic acid, stearic acid, fatty acid triglyceride, lecithin, choline, and carnitine; preferably, the ethylenediaminetetraacetic acid salt is selected from the group consisting of disodium ethylenediaminetetraacetate, dipotassium ethylenediaminetetraacetate, trisodium ethylenediaminetetraacetate, and tetrasodium ethylenediaminetetraacetate.

15. The polypeptide composition according to any one of claims 1 to 14, wherein the polypeptide composition further comprises at least one emulsifier.

16. The polypeptide composition according to claim 15, wherein the emulsifier is selected from the group consisting of any one or more of an ionic surfactant, a nonionic surfactant, and a zwitterionic surfactant;
preferably, the emulsifier is selected from the group consisting of any one or more of ammonium lauryl sulfate, N-dodecyl-β-D-maltoside, tridecyl-β-D-maltoside, sodium dodecyl sulfate, docusate sodium, cyclodextrin or a derivative thereof, chitosan or a derivative thereof, sodium polyoxyethylene alkyl sulfate, sodium laureth sulfate, sodium dioctyl sulfosuccinate, poloxamer, glyceryl monostearate, glyceryl monolaurate, sorbitan monolaurate, sorbitan monostearate, polyoxyethylene sorbitan fatty acid ester, sorbitan fatty acid ester, polyoxyethylene monopalmitate, polyoxyethylene hydrogenated castor oil, C₆-C₁₂ fatty acid triglyceride, and C₈-C₁₀ fatty acid triglyceride;
preferably, the at least one surfactant is selected from Tween; preferably, the at least one surfactant is selected from the group consisting of any one or more of Tween 20, Tween 40, Tween 60, Tween 65, Tween 80, and Tween 85.

17. The polypeptide composition according to any one of claims 1 to 16, wherein the polypeptide composition comprises by mass fraction:
a polypeptide: 0.5-10%,
fish oil: 3.0-50.0%,
an isolated BBI: 1.0-25.0%,
an isolated KTI: 1.0-20.0%,
absorption enhancer: 5.0-45.0%, and
emulsifier: 8.0-60.0%;
or
a polypeptide: 0.9-4.6%,
fish oil: 5.5-34.9%,
an isolated BBI: 2.9-10.0%,
an isolated KTI: 2.5-14.3%,
absorption enhancer: 12.9-36.1%, and
emulsifier: 14.9-49.6%;
or
a polypeptide: 0.9-4.6%,
fish oil: 11.9-34.9%,
an isolated BBI: 2.9-10.0%,
an isolated KTI: 2.5-14.3%,
absorption enhancer: 12.9-36.1%, and
emulsifier: 14.9-34.5%;
or
a polypeptide: 0.9-4.6%,
fish oil: 34.5-34.9%,
an isolated BBI: 2.9-7.5%,
an isolated KTI: 2.5-14.3%,
absorption enhancer: 12.9-35.6%, and
emulsifier: 14.9-34.5%;
or
a polypeptide: 0.9-1.8%,
fish oil: 11.9-34.5%,
an isolated BBI: 2.9-10.0%,
an isolated KTI: 12.6-14.3%,
absorption enhancer: 12.9-36.1%, and
emulsifier: 27.6-34.5%;
or
a polypeptide: 1.8-4.6%,
fish oil: 11.9-34.9%,
an isolated BBI: 7.5-10.0%,
an isolated KTI: 2.5-12.6%,
absorption enhancer: 35.6-36.1%, and
emulsifier: 14.9-27.6%.

18. The polypeptide composition according to any one of claims 1 to 17, wherein the polypeptide composition comprises by mass fraction:
a recombinant human insulin: 0.5-10%,
fish oil: 3.0-50.0%,
an isolated BBI: 1.0-25.0%,
an isolated KTI: 1.0-20.0%,
ethylenediaminetetraacetic acid or a salt thereof: 5.0-45.0%, and
Tween: 8.0-60.0%;
or
a recombinant human insulin: 0.9-4.6%,
fish oil: 5.5-34.9%,
an isolated BBI: 2.9-10.0%,
an isolated KTI: 2.5-14.3%,
ethylenediaminetetraacetic acid or a salt thereof: 12.9-36.1%, and
Tween: 14.9-49.6%;
or
a recombinant human insulin: 0.9-4.6%,
fish oil: 11.9-34.9%,
an isolated BBI: 2.9-10.0%,
an isolated KTI: 2.5-14.3%,
ethylenediaminetetraacetic acid or a salt thereof: 12.9-36.1%, and
Tween: 14.9-34.5%;
or
a recombinant human insulin: 0.9-4.6%,
fish oil: 34.5-34.9%,
an isolated BBI: 2.9-7.5%,
an isolated KTI: 2.5-14.3%,
ethylenediaminetetraacetic acid or a salt thereof: 12.9-35.6%, and
Tween: 14.9-34.5%;
or
a recombinant human insulin: 0.9-1.8%,
fish oil: 11.9-34.5%,
an isolated BBI: 2.9-10.0%,
an isolated KTI: 12.6-14.3%,
ethylenediaminetetraacetic acid or a salt thereof: 12.9-36.1%, and
Tween: 27.6-34.5%;
or
a recombinant human insulin: 1.8-4.6%,
fish oil: 11.9-34.9%,
an isolated BBI: 7.5-10.0%,
an isolated KTI: 2.5-12.6%,
ethylenediaminetetraacetic acid or a salt thereof: 35.6-36.1%, and
Tween: 14.9-27.6%.

19. The polypeptide composition according to any one of claims 1 to 18, wherein the polypeptide composition comprises in a unit dosage form:
a recombinant human insulin: 8-32 mg,
fish oil: 50-320 mg,
an isolated BBI: 26.7-88.9 mg,
an isolated KTI: 17.5-133.3 mg,
ethylenediaminetetraacetic acid or a salt thereof: 120-320 mg, and
Tween: 105-450 mg;
or
a recombinant human insulin: 8-32 mg,
fish oil: 105-320 mg,
an isolated BBI: 26.7-88.9 mg,
an isolated KTI: 17.5-133.3 mg,
ethylenediaminetetraacetic acid or a salt thereof: 120-320 mg, and
Tween: 105-320 mg;
or
a recombinant human insulin: 8-32 mg,
fish oil: 245-320 mg,
an isolated BBI: 26.7-52.5 mg,
an isolated KTI: 17.5-133.5 mg,
ethylenediaminetetraacetic acid or a salt thereof: 120-250 mg, and
Tween: 105-320 mg;
or
a recombinant human insulin: 8-16 mg,
fish oil: 105-320 mg,
an isolated BBI: 26.7-88.9 mg,
an isolated KTI: 111.1-133.3 mg,
ethylenediaminetetraacetic acid or a salt thereof: 120-320 mg, and
Tween: 245-320 mg;
or
a recombinant human insulin: 8 mg,
fish oil: 50-320 mg,
an isolated BBI: 26.7-37.5 mg,
an isolated KTI: 62.5-133.3 mg,
ethylenediaminetetraacetic acid or a salt thereof: 120-300 mg, and
Tween: 320-450 mg;
or
a recombinant human insulin: 16-32 mg,
fish oil: 105-245 mg,
an isolated BBI: 52.5-88.9 mg,
an isolated KTI: 17.5-111.1 mg,
ethylenediaminetetraacetic acid or a salt thereof: 250-320 mg, and
Tween: 105-245 mg;
or
a recombinant human insulin: 8-16 mg,
fish oil: 50-105 mg,
an isolated BBI: 37.5-88.9 mg,
an isolated KTI: 62.5-111.1 mg,
ethylenediaminetetraacetic acid or a salt thereof: 300-320 mg, and
Tween: 245-450 mg.

20. The polypeptide composition according to any one of claims 1 to 19, wherein the polypeptide composition comprises in a unit dosage form:
a recombinant human insulin: 8 mg,
fish oil: 320 mg,
an isolated BBI: 26.7 mg,
an isolated KTI: 133.3 mg,
ethylenediaminetetraacetic acid or a salt thereof: 120 mg, and
Tween: 320 mg;
or
a recombinant human insulin: 16 mg,
fish oil: 105 mg,
an isolated BBI: 88.9 mg,
an isolated KTI: 111.1 mg,
ethylenediaminetetraacetic acid or a salt thereof: 320 mg, and
Tween: 245 mg;
or
a recombinant human insulin: 32 mg,
fish oil: 245 mg,
an isolated BBI: 52.5 mg,
an isolated KTI: 17.5 mg,
ethylenediaminetetraacetic acid or a salt thereof: 250 mg, and
Tween: 105 mg;
or
a recombinant human insulin: 8 mg,
fish oil: 50 mg,
an isolated BBI: 37.5 mg,
an isolated KTI: 62.5 mg,
ethylenediaminetetraacetic acid or a salt thereof: 300 mg, and
Tween: 450 mg.

21. The polypeptide composition according to any one of claims 1 to 20, wherein the isolated BBI and the isolated KTI in the polypeptide composition are mixed in a certain ratio, so that an activity ratio of a total trypsin inhibitory activity (total TI) to a total chymotrypsin inhibitory activity (total CI) of the composition is from 0.5:1 to 10:1; preferably from 0.7:1 to 10:1; preferably from 0.91:1 to 6:1; preferably from 0.91:1 to 5.27:1; and the polypeptide composition comprises by mass fraction:
a recombinant human insulin: 0.5-10%,
fish oil: 3.0-50.0%,
an isolated BBI: 1.0-25.0%,
an isolated KTI: 1.0-20.0%,
ethylenediaminetetraacetic acid or a salt thereof: 5.0-45.0%, and
Tween: 8.0-60.0%;
or
a recombinant human insulin: 0.9-4.6%,
fish oil: 5.5-34.9%,
an isolated BBI: 2.9-10.0%,
an isolated KTI: 2.5-14.3%,
ethylenediaminetetraacetic acid or a salt thereof: 12.9-36.1%, and
Tween: 14.9-49.6%;
or
a recombinant human insulin: 0.9-4.6%,
fish oil: 11.9-34.9%,
an isolated BBI: 2.9-10.0%,
an isolated KTI: 2.5-14.3%,
ethylenediaminetetraacetic acid or a salt thereof: 12.9-36.1%, and
Tween: 14.9-34.5%;
or
a recombinant human insulin: 0.9-4.6%,
fish oil: 34.5-34.9%,
an isolated BBI: 2.9-7.5%,
an isolated KTI: 2.5-14.3%,
ethylenediaminetetraacetic acid or a salt thereof: 12.9-35.6%, and
Tween: 14.9-34.5%;
or
a recombinant human insulin: 0.9-1.8%,
fish oil: 11.9-34.5%,
an isolated BBI: 2.9-10.0%,
an isolated KTI: 12.6-14.3%,
ethylenediaminetetraacetic acid or a salt thereof: 12.9-36.1%, and
Tween: 27.6-34.5%;
or
a recombinant human insulin: 1.8-4.6%,
fish oil: 11.9-34.9%,
an isolated BBI: 7.5-10.0%,
an isolated KTI: 2.5-12.6%,
ethylenediaminetetraacetic acid or a salt thereof: 35.6-36.1%, and
Tween: 14.9-27.6%.

22. The polypeptide composition according to any one of claims 1 to 21, wherein a mass ratio of the isolated BBI to the isolated KTI in the polypeptide composition is from 0.1:1 to 5:1; preferably, the mass ratio of the isolated BBI to the isolated KTI is from 0.2:1 to 3:1; optionally, the mass ratio of the isolated BBI to the isolated KTI is 0.1:1, 0.2:1, 0.3:1, 0.4:1, 0.5:1, 0.6:1, 0.7:1, 0.8:1, 0.9:1, 1:1, 1.5:1, 2:1, 2.5:1, 3:1, 3.5:1, 4:1, 4.5:1, 5:1, or any numerical value between any two of the ratios.

23. The polypeptide composition according to any one of claims 16 to 22, wherein a mass ratio of the fish oil to Tween in the polypeptide composition is (10-70):(10-90), optionally, the mass ratio of the fish oil to Tween is (10-70):(10-70), (10-70):(10-30), 1:(0.43-9), 1:(0.43-2.33), 1:(0.43-1), 50:50, or 30:70.

24. A drug, wherein the drug comprises the polypeptide composition according to any one of claims 1 to 23;
preferably, the drug further comprises a pharmaceutically acceptable excipient.

25. The drug according to claim 24, wherein the pharmaceutically acceptable excipient comprises a coating, preferably, the coating comprises a gastric-soluble coating and/or an enteric-soluble coating;
preferably, the pharmaceutically acceptable excipient comprises any one or more of hydroxypropyl cellulose, hydroxypropyl methylcellulose, acrylic resin, polyvinylpyrrolidone, cellulose acetate phthalate, hydroxypropyl methylcellulose phthalate, and hydroxypropyl methylcellulose acetate succinate;
preferably, the pharmaceutically acceptable excipient is selected from the group consisting of any one or more of a diluent, a disintegrant, a binder, a glidant, a flavoring agent, and a lubricant;
preferably, the excipient is selected from the group consisting of any one or more of lactose, sorbitol, xylitol, mannitol, calcium sulfate, calcium carbonate, calcium hydrogen phosphate, microcrystalline cellulose, silicified microcrystalline cellulose, starch, pregelatinized starch, a lactose-starch complex, a lactose-cellulose complex, and a mannitol-starch complex;
preferably, the disintegrant is selected from the group consisting of any one or more of croscarmellose sodium, cross-linked polyvinylpyrrolidone, sodium starch glycolate, cross-linked sodium starch glycolate, and low-substituted hydroxypropyl cellulose;
preferably, the binder is selected from the group consisting of any one or more of starch paste, gelatin solution, sucrose solution, methylcellulose, ethylcellulose, sodium carboxymethyl cellulose, hydroxypropyl methylcellulose, hydroxypropyl cellulose, and polyvinylpyrrolidone;
preferably, the lubricant is selected from the group consisting of any one or more of sodium stearyl fumarate, magnesium lauryl sulfate, polyethylene glycol, magnesium dodecyl sulfate, sodium dodecyl sulfate, magnesium stearate, calcium stearate, micronized silica gel, and talc;
preferably, the flavoring agent is selected from the group consisting of any one or more of mannitol, sorbitol, aspartame, stevioside, sucralose, aspartame, neotame, steviol glycosides, acesulfame chloride, and flavoring essence.

26. The polypeptide composition according to any one of claims 1 to 23, or the drug according to claim 24 or 25, wherein the polypeptide composition or the drug is present in a dosage form selected from the group consisting of: a tablet, a pill, a capsule, an emulsion, a syrup, or a suspension; preferably, the polypeptide in the capsule is in a suspended state.

27. The polypeptide composition according to any one of claims 1 to 23, or the drug according to claim 24 or 25, wherein the polypeptide composition or the drug is anhydrous.

28. The polypeptide composition according to any one of claims 1 to 23, or the drug according to claim 24 or 25, wherein the polypeptide composition or the drug is an oral or injectable dosage form.

29. Use of the polypeptide composition according to any one of claims 1 to 23, or the drug according to claim 24 or 25 in the preparation of an oral drug for treating or preventing a disease, wherein the disease is selected from the group consisting of hyperglycemia, type 1 diabetes, type 2 diabetes, non-insulin-dependent diabetes, maturity-onset diabetes of the young, gestational diabetes, obesity, non-alcoholic fatty liver disease, hepatic steatosis, hepatitis, liver fibrosis, cirrhosis, liver cancer, Alzheimer's disease, Parkinson's disease, and diseases with impaired glucose tolerance.

30. A method for treating or preventing a disease, comprising orally administering to a subject in need thereof a therapeutically effective amount of the polypeptide composition according to any one of claims 1 to 23, or the drug according to claim 24 or 25, wherein the disease is selected from the group consisting of hyperglycemia, type 1 diabetes, type 2 diabetes, non-insulin-dependent diabetes, maturity-onset diabetes of the young, gestational diabetes, obesity, non-alcoholic fatty liver disease, hepatic steatosis, hepatitis, liver fibrosis, cirrhosis, liver cancer, Alzheimer's disease, Parkinson's disease, and diseases with impaired glucose tolerance.

31. The method for treating or preventing a disease according to claim 30, wherein the method comprises orally administering to a subject in need thereof the polypeptide composition according to any one of claims 1 to 23, or the drug according to any one of claims 24 to 25, at a single dose of 8-50 mg of the polypeptide, once, twice, or three times daily.

32. The method for treating or preventing a disease according to claim 30, wherein the method comprises orally administering to a subject in need thereof the polypeptide composition according to any one of claims 1 to 23, or the drug according to any one of claims 24 to 25, at a single dose of 8 mg, 24 mg, or 50 mg of the polypeptide, once, twice, or three times daily.
